# NEW EUROPEAN PATENT SPECIFICATION

(11) **EP 0 930 296 B2**
(45) Date of publication and mention of the opposition decision: **02.11.2005**
(45) Mention of the grant of the patent: 24.07.2002
(21) Application number: 97940373.0
(22) Date of filing: 12.09.1997
(51) Int. Cl.: C07C 405/00, C07D 313/00, A61K 31/557, A61K 31/335

(54) **FLUORINATED PROSTAGLANDIN DERIVATIVES AND MEDICINES**
FLUORIERTE PROSTAGLANDINDERIVATE UND MEDIKAMENTE
DERIVES DE PROSTAGLANDINE FLUORES ET MEDICAMENTS

(30) Priority: 17.09.1996 JP 24515596; 25.12.1996 JP 34628696; 13.01.1997 JP 410997
(43) Date of publication of application: 21.07.1999
(73) Proprietor: ASAHI GLASS COMPANY LTD., Tokyo 100-8405 (JP); SANTEN PHARMACEUTICAL CO., LTD., Higashiyodogawa-ku, Osaka-shi, Osaka 533-8651 (JP)
(72) Inventor: SHIRASAWA, Eiichi, Santen Pharmaceutical Co. Ltd., Ikoma-shi, Nara 630-01 (JP); KAGEYAMA, Masaaki, Santen Pharmaceutical Co. Ltd., Ikoma-shi, Nara 630-01 (JP); MORI, Nobuaki, Asahi Glass Company Ltd., Yokohama-shi, Kanagawa 221 (JP); SAKATA, Kazuhisa, Asahi Glass Company Ltd., Yokohama-shi, Kanagawa 221 (JP); NAKANO, Takashi, Asahi Glass Company Ltd., Yokohama-shi, Kanagawa 221 (JP); MATSUMURA, Yasushi, Asahi Glass Company Ltd., Yokohama-shi, Kanagawa 221 (JP); MORIZAWA, Yoshitomi, Asahi Glass Company Ltd., Yokohama-shi, Kanagawa 221 (JP)
(74) Representative: Perrey, Ralf
(86) International application number: PCT/JP1997/003236
(87) International publication number: WO 1998/012175

(56) References cited:
- EP-A- 0 850 926
- EP-B1- 0 364 417
- EP-B1- 0 938 472
- WO-A-98/21181
- WO-A-99/12898
- JP-A- 7 070 054
- JP-A- 7 165 703
- JP-A- 24 515 596
- JP-T- 3 501 025
- BIOORG. KHIM., Vol. 7, No. 3, (1981), BEZUGLOV V.V. et al., "Synthesis of Tritium-Labeled 15-Fluoro-15-Deoxyprostaglandins A2, B2 and F2Alpha", pages 448-454.
- B.Resul et al, "Structure-Activity Relationships of Prostaglandin Analogues as Ocular Hypotensive Agents", Current Opinion in Therapeutic Patents, June 1993, 781-795
- SCRIP Extract: "First approval for latanoprost in US": SCRIP 2137, p. 21, 5 July 1996
- J. Stjernschantz et al, "Phenyl substituted prostaglandin analogues for glaucoma treatment", Drugs of the Future, 1992, 17(8), 691-704
- W. E. Barnette, "The synthesis and biology of fluorinated prostacyclins", Critical Reviews in Biochemistry, 15(3), 201-35, 1984

## Description

The present invention relates to fluorine-containing prostaglandin derivatives having a fluorine atom at the 15-position and their salts and medicines containing them as an active ingredient, particularly, preventive or therapeutic medicines for eye diseases.

The naturally occurring prostaglandins (PGs) are a class of biologically active substances synthesized in the body and control cellular functions in various tissues of the body as local hormones having various biological activities. The PGFs, a group of naturally occurring PGs, which have a uterotonic action, a sterilizing action and a sex cycle synchronizing action, are used as parturifacients and sex cycle synchronizers for animals. Therefore, research for development of PGF derivatives which are more effective as these drugs but do not have side effects is extensively conducted both at home and abroad.

Because prostaglandins generally have various biological activities, in order to obtain derivatives selectively showing a desired action, it is necessary to tactically utilize differences in receptor selectivity made by subtle structural change. The present inventors focused on the ω-chain of PG and supposed that the position, species and number of the substituents on the terminal aryl group or heteroaromatic ring appreciably influence the selectivity. Further, they also speculated that the fat-solubility of a compound largely contributed to its absorbability into the body and pharmacological actions and that in the case of a carboxylic acid derivative, the rate of hydrolysis of its ester in vivo affected the durability.

On the other hand, the PGFs, a group of naturally occurring PGs, are known to lower intraocular pressure when topically applied to the eye and are expected to find applications as therapeutic medicines for ocular hypertension or glaucoma (USP 4,599,353). However, they are irritant to the eye and have a problem of their inflammatory side effects such as congestion and damage to the cornea. Therefore, research for development of PGF derivatives which do not have such side effects is extensively conducted both at home and abroad. PGF derivatives having a cyclic structure in the ω-chain are also known. Shielnshantz et al. reported specific PGA, PGB, PGD, PGE and PGF derivatives modified through introduction of a cyclic structure are less irritant and congestive to the eye (JP-A-8-109132). However, the irritation of the eye caused by them is not weak enough.

Ophthalmic compositions for local therapeutic medicines for glaucoma and ocular hypertension containing a chloprostenol or fluprostenol analog have been also reported (JP-A-7-165703). A typical compound disclosed in this literature, 13,14-dihydro-17-phenyl-18,19,20-trinor PGF_{2α} isopropyl ester (Latanoprost) is reported to show strong effect of lowering intraocular pressure and hardly congest the conjunctiva. However, there is still plenty of room for improvement.

Bezglov et al. reported 15-fluoro-15-deoxy PGF_{2α}, which is derived from naturally occurring PGF_{2α} by introducing fluorine at the 15-position and retains the skeleton of its origin. 15-Fluoro-15-deoxy PGF_{2α} is reported to have remarkable pharmacological actions such as the 100-fold greater contraction action and the 1000-fold relaxation action on smooth muscle in the respiratory system as compared with those of the naturally occurring PGF_{2α} and the action on the smooth muscle in the digestive and circulatory systems comparable to that of the naturally occurring PGF_{2α} (Izv. Akad. Nauk SSSR, Ser. Biol., 6, 831 (1989)). Furthermore, Bezuglov et al., in Bioorg. Khim. 7 (3), 448-454 (1981), describe the synthesis of tritium-labeled 15-fluoro-15-deoxyprostaglandins A₂, B₂ and F_{2α} (as well as 15-fluoro-15-deoxyprostaglandin F_{2α} methyl ester) and tritium-labeled prostaglandin F_{2α} by the reaction of heterologous catalytic isotope exchange with gaseous tritium in solution. However, no report has been made on any pharmacological actions of the compound on any eye disease, particularly on glaucoma.

No prostaglandin F derivatives that have a fluorine atom at the 15-position have been known except 15-fluoro-15-deoxy-PGF_{2α} and 15-fluoro-15-deoxy-PGF_{2α} methyl ester. Especially, no report has been made on 15,15-difluoro-15-deoxy F_{2α} derivatives having synthetic side chains per se or their synthesis. In the above-mentioned literature, 15-fluoro-15-deoxy PGF_{2α} is synthesized by a fairly impractical method comprising fluorination of the 15-position of naturally occurring PGA₂ and subsequent conversion into 15-fluoro-15-deoxy PGE₂ followed by conversion into 15-fluoro-15-deoxy PGF_{2α} through reduction of the carbonyl group at the 9-position (Dokl. Akad. Nauk SSSR, 250,468 (1980)). The present inventors took a new approach to synthesize 15-fluoro-15-deoxy PGF_{2α} derivatives having synthetic side chains and studied their pharmacological actions on eye diseases as well as those of 15-fluoro-15-deoxy PGF_{2α}.

Because prostaglandins generally have various biological activities, in order to obtain derivatives selectively showing a desired action, it is necessary to tactically utilize differences in receptor selectivity made by subtle structural change. The present inventors focused on the ω-chain of PG and supposed that the position, species and number of the substituents on the terminal aryl group or heteroaromatic ring appreciably influence the selectivity. Further, they also speculated that the fat-solubility of a compound largely contributed to its absorbability into the eye and effect of lowering intraocular pressure after application to the eye and that in the case of a carboxylic acid derivative, the rate of hydrolysis of its ester in vivo affected the durability.

### DISCLOSURE OF THE INVENTION

The present inventors measured biological activities of 15-fluoro-15-deoxy PGF_{2α} and its derivatives to access their usefulness as medicines. The present inventors also measured the biological activities of derivatives of 15-fluoro-15-deoxy PGF_{2α} which had an aryloxy group in the ω-chain and were prepared by modifying the carboxyl group or the hydroxyl group of the prostaglandin to access their usefulness as medicines. As a result, the present inventors have found that 15-fluoro-15-deoxy PGF_{2α} and its derivatives are not only comparable to the naturally occurring prostaglandins F in the effect of lowering intraocular pressure but also better in respect of irritation of the eye and influences on ocular tissues such as the cornea, the iris and the conjunctive and are excellent in durability. The present invention relates to the novel 15-fluoro-15-deoxy PGF_{2α} derivatives, medicines containing the derivatives as an active ingredient and medical use of 15-fluoro-15-deoxy PGF_{2α} and its derivatives for eye diseases, and provides the following.

A fluorine-containing prostaglandin derivative of the following formula (1) (exclusive of 15-fluoro-15-deoxy PGF_{2α} and 15-fluoro-15-deoxy-PGF_{2α} methyl ester) or a salt thereof, and a medicine containing it as an active ingredient.

Said fluorine-containing prostaglandin derivative has the following formula (1) : 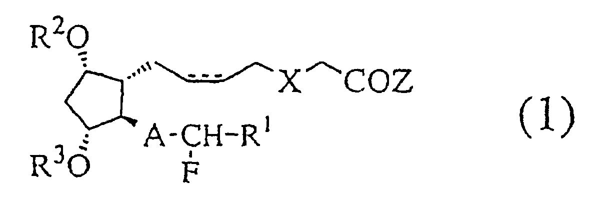 wherein A is an ethylene group, a vinylene group, an ethynylene group, -OCH₂- or -SCH₂-,
R¹ is a substituted or unsubstituted C₃₋₈ alkyl group, a substituted or unsubstituted C₃₋₈ alkenyl group, a substituted or unsubstituted C₃₋₈ alkynyl group, a substituted or unsubstituted C₃₋₈ cycloalkyl group, a substituted or unsubstituted aralkyl group or a substituted or unsubstituted aryloxyalkyl group,
each of R² and R³ which are independent of each other, is a hydrogen atom or an acyl group, or forms a single bond as described later,
X is -CH₂-, -O- or -S-,
Z is -OR⁴ , -NHCOR⁵, -NHSO₂R⁶ or -SR⁷, or forms a single bond together with R² or R³,
each of R⁴, R⁵, R⁶ and R⁷ which are independent of one another, is a hydrogen atom, an alkyl group, an alkenyl group, an alkynyl group, a cycloalkyl group, an aryl group or an aralkyl group,
and a dual line consisting of solid and broken lines is a single bond, a cis-double bond or a trans-double bond.

Among the fluorine-containing prostaglandin derivatives of the above formula (1), those wherein at least one of R¹ and R² is an acyl group, or Z is -OR⁴ (provided that R⁴ is other than a hydrogen atom), -NHCOR⁵, -NHSO₂R⁶ or -SR⁷ are preferable in view of biological activities on eye diseases and physical properties.

In the following description, the term "lower" for an organic group corresponds to a carbon number of from 1 to 6. A preferred lower organic group is an organic group having from 1 to 4 carbon atoms. An "alkyl group" may be linear or branched, and unless otherwise noted, a lower alkyl group is preferred. Specific examples include a methyl group, an ethyl group, a propyl group, an isopropyl group, a butyl group, an isobutyl group, a t-butyl group, a pentyl group and a hexyl group. An "alkenyl group" is preferably a lower alkenyl group, unless otherwise noted, and more preferably a linear or branched alkenyl group having from 2 to 6 carbon atoms and one unsaturated group. Specific examples include a vinyl group, an allyl group, a 1-propenyl group, an isopropenyl group, a 3-butenyl group, a 3-pentenyl group and a 4-hexenyl group.

An "alkynyl group" is preferably a lower alkynyl group, unless otherwise noted, more preferably a linear or branched alkynyl group having from 2 to 6 carbon atoms and one unsaturated group. Specific examples include a 1-propynyl group, a 2-propynyl group, a 3-butynyl group, a 3-pentynyl group and a 4-hexynyl group. As an "alkoxy group", although a wide variety of common alkoxy groups may be used, a lower alkoxy group is preferred, and more preferred is a linear or branched alkoxy group having from 1 to 4 carbon atoms. Specific examples include a methoxy group, an ethoxy group, a propoxy group and a butoxy group.

A "halogen atom" means a fluorine atom, a chlorine atom, a bromine atom or an iodine atom. An "aryl group" means a monovalent aromatic hydrocarbon group which may have a substituent (such as a lower alkyl group, a halogen atom, a lower alkoxy group or a lower alkylamino group), preferably a phenyl group or its derivative. For example, a phenyl group, a tolyl group, a halophenyl group (such as a chlorophenyl group, a fluorophenyl group or a bromophenyl group), a dihalophenyl group (such as a dichlorophenyl group, a difluorophenyl group or a dibromophenyl group), a trihalophenyl group (such as a trichlorophenyl group, a trifluorophenyl group or a tribromophenyl group) or an alkoxyphenyl group (such as a methoxyphenyl group or an ethoxyphenyl group) may be mentioned. An "aralkyl group" means an aryl-substituted alkyl group, in which the aryl group as the substituent may be such an aryl group as described above, and the carbon number of the alkyl group is preferably from 1 to 4. Specific examples include a benzyl group, a benzhydryl group, a trityl group and a phenethyl group.

A "cycloalkyl group" means an unsubstituted or substituted 3 to 8-membered cycloalkyl group, and when substituted, may have a lower alkyl group, a halogen atom or an alkoxy group as a substituent. For example, a cyclopropyl group, a cyclobutyl group, a cyclopentyl group, a cyclohexyl group, a cycloheptyl group, a methylcyclohexyl group, a dimethylcyclopentyl group, a dimethylcyclohexyl group, a chlorocyclohexyl group or a dichlorocyclohexyl group may be mentioned.

A "haloalkyl group" means a lower haloalkyl group having at least one halogen atom. A fluoromethyl group, a difluoromethyl group, a trifluoromethyl group, a trifluoroethyl group, a pentafluoroethyl group, a chloromethyl group, a dichloromethyl group, a trichloromethyl group or a bromomethyl group may be mentioned.

An "acyl group" means a monovalent or polyvalent group derived from a carboxylic acid by removing hydroxyl group(s) from all the carboxyl group(s). As the carboxylic acid, a saturated or unsaturated aliphatic carboxylic acid, a carbocyclic carboxylic acid or a heterocyclic carboxylic acid may be mentioned. As the carbocyclic carboxylic acid, a saturated or unsaturated alicyclic carboxylic acid or an aromatic carboxylic acid may be mentioned.

Among the fluorine-containing prostaglandin derivatives of the formula (1) (hereinafter referred to as the fluorine-containing prostaglandin derivatives (1)), the following compounds are preferred from the standpoint of biological activities and physical properties.

As A, a vinylene group or an ethylene group is preferred, and a vinylene group is particularly preferred. The vinylene group includes cis- or trans-vinylene groups. A trans-vinylene group is particularly preferred.

As X, -CH₂- is particularly preferred.

The dual line consisting of solid and broken lines is preferably a cis-double bond.

R¹ is preferably an organic group corresponding to the ω-chain moiety of the naturally occurring PGF_{2α} (when the rest is not of the naturally occurring type) or an organic group corresponding to the ω-chain moiety of any of various synthetic PGs F_{2α}. Such organic groups include, for example, a C₃₋₈ alkyl group, a C₃₋₈ alkenyl group, a C₃₋₈ alkynyl group, a C₃₋₈ cycloalkyl group, an aralkyl group, an aryloxy group having an aryl group such as a phenyl group, and such groups having various substituents.

The alkyl group may have a cyclic organic group such as a cycloalkyl group as a substituent, and the alkenyl group and the alkynyl group may have a cyclic organic group such as an aryl group or a cycloalkyl group as a substituent. For example, R¹ may be a cycloalkyl group-substituted alkyl group, a cycloalkyl group-substituted alkenyl group, or an aryl group-substituted alkenyl group. Further, it may be an organic group having an oxygen atom or a sulfur atom introduced to replace a carbon atom of a linear organic group such as an alkyl group, or an organic group having a cyclic organic group such as a cycloalkylene group or an arylene group introduced between two carbon atoms of a linear organic group. Further, a cycloalkyl group, an aralkyl group, an aryloxy group and an organic group having such a group as a substituent may have a linear organic group such as an alkyl group as a substituent on the ring moiety. Substituents in R¹ include, in addition to the above-mentioned substituents, a halogen atom, an oxygen atom-containing substituent, a sulfur atom-containing substituent, a nitrogen atom-containing substituent, and others.

When R¹ is a linear substituted or unsubstituted group, a linear C₅₋₆ alkyl group, a linear C₅₋₆ alkenyl group and a linear C₅₋₆ alkynyl group and such groups substituted with one or two methyl group are particularly preferred. Specific linear groups as R¹ include the following groups. Among them, preferred are a n-pentyl group, a 2-methylhexyl group, a 1-methyl-3-pentynyl group, a 1-methyl-3-hexynyl group, and a 1,1-dimethyl-3-hexynyl group.

A n-propyl group, a n-butyl group, a n-pentyl group, a n-hexyl group, a n-heptyl group, a n-octyl group, a n-decyl group, a 1-methylpentyl group, a 1,1-dimethylpentyl group, a 1-methylhexyl group, a 2-methylpentyl group, a 2-methylhexyl group, a 3-pentenyl group, a 1-methyl-3-pentenyl group, a 1-methyl-3-hexenyl group, a 1,1-dimethyl-3-pentenyl group, a 1,1-dimethyl-3-hexenyl group, a 2-methyl-3-pentenyl group, a 2-methyl-3-hexenyl group, a 3-pentynyl group, a 1-methyl-3-pentynyl group, a 1-methyl-3-hexynyl group, a 2-methyl-3-pentynyl group, a 2-methyl-3-hexynyl group, a 1,1-dimethyl-3-pentynyl group, and a 1,1-dimethyl-3-hexynyl group.

The substituted or unsubstituted cycloalkyl group as R¹ is preferably a C₃₋₈ cycloalkyl group, or such a cycloalkyl group substituted by at least one lower alkyl group. Particularly preferred is an unsubstituted cyclopentyl group, an unsubstituted cyclohexyl group, a C₁₋₄ alkyl group-substituted cyclopentyl group, or a C₁₋₄ alkyl group-substituted cyclohexyl group.

The substituted or unsubstituted aralkyl group as R¹ is preferably an aralkyl group which contains, for example, a benzene ring, a furan ring, a thiophene ring or a naphthalene ring and is substituted by, for example, a halogen atom, a haloalkyl group, an alkoxy group or a hydroxyl group. The carbon number of the alkyl moiety (i.e. the alkylene group) of the aralkyl group is preferably from 1 to 4. A particularly preferred aralkyl group is a C₁₋₂ alkyl group substituted with a phenyl group or a C₁₋₂ alkyl group substituted with a phenyl group substituted with one or two lower alkyl groups or halogen atoms. Specifically, a phenylmethyl group, a 2-phenylethyl group, a 3-methylphenylmethyl group, a 2-(3-methylphenyl)ethyl group, a 3-trifluoromethylphenylmethyl group, a 2-(3-trifluoromethylphenyl)ethyl group, a 3-chlorophenylmethyl group, a 2-(3-chlorophenyl)ethyl group, a 2-(3,5-dichlorophenyl)ethyl group and a 2-(3,4-dichlorophenyl) ethyl group are preferred.

The substituted or unsubstituted aryloxyalkyl group as R¹ is preferably an aryloxyalkyl group which contains, for example, a benzene ring, a furan ring, a thiophene ring or a naphthalene ring and may have, for example, a halogen atom, a haloalkyl group, an alkoxy group or a hydroxyl group as a substituent on the aryl moiety. The aryl moiety is preferably a phenyl group which is substituted with from 1 to 3 halogen atoms or haloalkyl groups. The carbon number of the alkyl moiety substituted with an aryloxy group is preferably from 1 to 3. Specific preferred aryloxyalkyl groups are a phenoxymethyl group, a 3-chlorophenoxymethyl group, a 3-fluorophenoxymethyl group, a 3-trifluoromethylphenoxymethyl group, a 3,5-dichlorophenoxymethyl group, a 3,4-dichlorophenoxymethyl group, a 3,5-difluorophenoxymethyl group, a 3,4-difluorophenoxymethyl group, a 3,5-bis(trifluoromethyl)phenoxymethyl group and a 3,4-bis(trifluoromethyl)phenoxymethyl group.

As R¹, in addition to those described above, a C₁₋₄ alkyl group substituted by the above-mentioned cycloalkyl group is preferred as a type of substituted alkyl group. As such a cycloalkyl group, a cyclopentyl group or a cyclohexyl group is preferred, and as such an alkyl group, a C₁₋₂ alkyl group is preferred. Specific examples include a cyclopentylmethyl group, a 2-cyclopentylethyl group and a cyclohexylmethyl group.

As R¹, more preferred are the above-mentioned substituted or unsubstituted aryloxyalkyl groups and substituted or unsubstituted aralkyl groups. Among them, a substituted phenoxymethyl group such as a 3,5-dichlorophenoxymethyl group, a 3,4-dichlorophenoxymethyl group or a 3-chlorophenoxymethyl group, a phenylmethyl group and a substituted phenylmethyl group such as a 3-methylphenylmethyl group, a 3-trifluoromethylphenylmethyl group, a 3,5-dichlorophenylmethyl group, a 3,4-dichlorophenylmethyl group are preferred. Particularly, a halogen atom-substituted phenoxymethyl group such as a 3,5-dichlorophenoxymethyl group, a 3,4-dichlorophenoxymethyl group or a 3-chlorophenoxymethyl group and a phenoxymethyl group are preferred.

Each of R² and R³ which are independent of each other, is a hydrogen atom or an acyl group, or forms a single bond as described later. It is preferred that both R² and R³ are hydrogen atoms, or that either R² or R³ is an acyl group and the other is a hydrogen atom. When only one of them is an acyl group, it is preferred that R² is an acyl group. Compounds wherein at least one of R² and R³ is an acyl group are useful as prodrugs because they can hydrolyze in vivo to biologically active compounds. As the acyl group, a C₂₋₂₀ acyl group, particularly, an aliphatic hydrocarbon type C₂₋₂₀ acyl group is preferred. In particular, fluorine-containing prostaglandin derivatives wherein either R² or R³ is an aliphatic linear hydrocarbon type acyl group having a carbon number of at least 4 are useful as prodrugs having improved fat solubility.

Z is -OR⁴, -NHCOR⁵, -NHSO₂R⁶, -SR⁷ or represents a single bond together with R² or R³, which means cyclization of a compound wherein Z is OH and either R² or R³ is a hydrogen atom (a compound having a carboxyl group at the end of the α-chain and a hydroxyl group either at the 9-position or at the 11-position) by esterification of the carboxyl group and the hydroxyl group to form an ester bond between the end of the α-chain and the 9- or 11-position. Such cyclic compounds having an ester bond can hydrolyze in vivo into biologically active compounds, and therefore are useful as prodrugs.

As R⁴ - R⁷ in the groups represented by -OR⁴, -NHCOR⁵, -NHSO₂R⁶ and -SR⁷, a hydrogen atom, an alkyl group, an alkenyl group, an alkynyl group, a cycloalkyl group, an aryl group and an aralkyl group may be mentioned. The alkyl group, the alkenyl group, the alkynyl group and the alkyl moiety of the aralkyl group may be linear or branched and may have various substituents such as halogen atoms. The cycloalkyl group, the aryl group and the aralkyl group may have an alkyl group or other substituents on the ring. As such substituents, the substituents described above for R¹ may be mentioned.

The alkyl group, the alkenyl group and the alkynyl group as R⁴ - R⁷ preferably have a carbon number of at most 20, particularly, at most 8. Specific examples of these linear hydrocarbon groups include a methyl group, an ethyl group, a n-propyl group, an isopropyl group, a n-butyl group, a n-pentyl group, a n-hexyl group, a n-heptyl group, a n-octyl group, a n-decyl group, a 1-methylpentyl group, a 1,1-dimethylpentyl group, a 1-methylhexyl group, a 2-methylpentyl group and 2-methylhexyl group. As the alkenyl group, an allyl group, a 2-butenyl group, a 3-pentenyl group, 1-methyl-3-pentenyl group, 1-methyl-3-hexenyl group, 1,1-dimethyl-3-pentenyl group and a 1,1-dimethyl-3-hexenyl group may be mentioned. As the alkenyl group, a propargyl group, a 3-pentynyl group, a 1-methyl-3-pentynyl group, a 1-methyl-3-hexynyl group, a 1,1-dimethyl-3-pentynyl group and a 1,1-dimethyl-3-hexynyl group may be mentioned.

As the substituted alkyl group as R⁴ - R⁷, a halogen atom-substituted alkyl group or a cycloalkyl group-substituted alkyl group may be mentioned. The carbon number of the halogen atom-substituted alkyl group is preferably at most 6, and the carbon number of the alkyl moiety of the cycloalkyl group-substituted alkyl group is preferably from 1 to 2. As the halogen atom-substituted alkyl group, for example, a trifluoromethyl group or a pentafluoroethyl group may be mentioned. As the cycloalkyl group-substituted alkyl group, for example, a cyclobutylmethyl group, a cyclopentylmethyl group or a cyclohexylmethyl group may be mentioned.

The carbon number of the cycloalkyl group as R⁴ - R⁷ is preferably at most 10. Specific examples include a cyclopropyl group, a cyclobutyl group, a cyclopentyl group, a cyclohexyl group, a 2,2-dimethylcyclopropyl group, a 3-cyclopentenyl group, a 3-cyclohexynyl group and a cyclooctanyl group.

As the aryl group as R⁴ - R⁷, a substituted or unsubstituted phenyl group is preferred. As the substituent, an alkyl group (preferably having a carbon number of at most 4), a halomethyl group, a halogen atom, an alkoxy group, an acyl group, an acylamino group or a nitro group is preferred. Specific examples include a phenyl group, a 4-methylphenyl group, a 4-(t-butyl)phenyl group, a 3-trifluoromethylphenyl group, a 4-trifluoromethylphenyl group, a 4-chlorophenyl group, a 4-acetylphenyl group, a 4-benzoylphenyl group, a4-acetylaminophenyl group, a 4-benzoylaminophenyl group, a 3-nitrophenyl group and a 4-nitrophenyl group.

As the aralkyl group as R⁴ - R⁷, an aralkyl group consisting of an alkyl moiety having a carbon number of at most 4 (preferably a carbon number of 1 or 2) and a phenyl group is preferred. The phenyl group may be substituted with an alkyl group (preferably having a carbon number of at most 4), a halomethyl group, a halogen atom, an alkoxy group, an acyl group, an acylamino group, a nitro group or the like. The alkyl moiety of the aralkyl group may be branched.

Specific examples include a benzyl group, a phenethyl group, a diphenylmethyl group, a (3-methylphenyl) methyl group, a (3-chlorophenyl)methyl group, a (3-fluoromethylphenyl)methyl group, a (3-bromophenyl)methyl group, a {(3-trifluoromethyl)phenyl}methyl group, a 1-(3-methylphenyl)ethyl group, a 1-(3-chlorophenyl)ethyl group, a 1-{(3-trifluoromethyl)phenyl)ethyl group, a 1-(3-fluorophenyl)ethyl group, a 1-(3-bromophenyl)ethyl group, a 2-(3-methylphenyl)ethyl group, a 2-(3-chlorophenyl)ethyl group, a 2-((3-trifluoromethyl)phenyl}ethyl group, a 2-(3-fluorophenyl)ethyl group, a 2-(3-bromophenyl) ethyl group, a 1-methyl-2-(3-methylphenyl)ethyl group, a 1-methyl-2-(3-chlorophenyl)ethyl group, a 1-methyl-2-{(3-trifluoromethyl)phenyl}ethyl group, a 1-methyl-2-(3-fluorophenyl)ethyl group and a 1-methyl-2-(3-bromophenyl)ethyl group.

Each of R⁴ - R⁷ is preferably an alkyl, cycloalkyl or aralkyl group which may have a substituent. As the substituent, a halogen atom or an alkyl group having a carbon number of at most 4 which is bonded to a ring is preferred. Particularly preferred R⁴ - R⁷ are alkyl groups, and a haloalkyl is particularly preferred as R⁶.

Z is preferably a group represented by -OR⁴. R⁴ in Z is preferably a hydrogen atom or a C₁₋₂₀ hydrocarbon group such as an alkyl group, a cycloalkyl group or an aralkyl group. Compounds wherein R⁴ is a hydrocarbon group are useful as prodrugs because they can hydrolyze in vivo into biologically active compounds. It is possible to improve the fat solubility of compounds by proper selection of hydrocarbon groups. As Z, particularly preferred are a hydroxyl group, an isopropoxy group, an ethoxy group, a methoxy group, an isobutoxy group, a cyclohexyloxy group and a benzyloxy group.

A fluorine-containing prostaglandin derivative of the present invention having an acidic group such as a carboxy group, for example like those wherein Z is a hydroxyl group, may take the form of a salt with a base. Similarly, when a compound of the present invention has a basic group such as an amino group, it may take the form of a salt with an acid. Salts with bases include alkali metal salts such as sodium salts and potassium salts, alkaline earth metal salts such as calcium salts and magnesium salts and ammonium salts such as unsubstituted ammonium salts and alkyl-substituted ammonium salts. Salts with acids include inorganic acid salts such as hydrochlorides, sulfates and phosphates and organic acid salts such as acetates, oxalates, citrates, succinates and p-toluenesulfonates.

The fluorine-containing prostaglandin derivatives of the present invention can be synthesized by forming the prostaglandin nucleus by a general method for synthesis of prostaglandin F_{2α} and then fluorinating the hydroxyl group at the 15-position into a fluorine atom. For synthesis of prostaglandin F_{2α}, known processes may be employed. For example, the prostaglandin F_{2α} nucleus can be synthesized by converting a Corey lactone as the starting material into an ω-chain-containing Corey lactone through introduction of the ω-chain, reducing the lactone to a lactol, introducing the α-chain unit through the Wittig reaction, converting the carboxyl moiety into an ester, an acylamide, a sulfonamide or a thioester, and optionally deprotecting or acylating a hydroxyl group, as a precursory prostaglandin to be fluorinated.

Then, the hydroxyl group at the 15-position of the precursory prostaglandin is fluorinated. Because fluorination of a hydroxyl group usually gives a stereochemically inverted fluorination product as the main product, a fluorination product having the naturally occurring configuration is produced efficiently from a precursor having a hydroxyl group of the opposite configuration. Fluorination of such a hydroxyl group to give a fluorine-containing prostaglandin derivative of the formula (1) is performed by various known fluorination methods, for example, by a method using various nucleophilic fluorinating agents in inert solvents.

When the precursory prostaglandin has a functional group liable to fluorinate during the fluorination, it is preferred to preliminarily protect the functional group by a protecting group. For example, a hydrogen atom as R² or R³ is preferably protected by a protecting group during the fluorination of the hydroxyl group at the 15-position and then the protection group is removed to give a fluorine-containing prostaglandin derivative of the present invention.

The protecting groups include, for example, a triorganosilyl group, an acyl group, an alkyl group, an aralkyl group and a cyclic ether group. A common protecting group for 1,3-diols such as a cyclic acetal, a cyclic ketal, a cyclic orthoester, a cyclic silyl ether, a cyclic carbonate and a cyclic boronate may also be used. An acyl group to protect a hydroxyl group at the 9- or 11-position may be the same as or different from the acyl group as R² or R³. A compound having an acyl group which is different from the acyl group used as the protecting group can be obtained by removing the protecting group and then introducing the different acyl group.

The triorganosilyl group is a group having three organic groups such as alkyl groups, aryl groups, aralkyl groups or alkoxyl groups bonded to a silicon atom. Particularly preferred is a triorganosilyl group having three groups of at least one kind selected from the group consisting of lower alkyl groups and aryl groups. Specifically, a t-butyldimethylsilyl group, a t-butyldiphenylsilyl group, a triethylsilyl group, a triphenylsilyl group or a triisopropylsilyl group may, for example, be preferred.

As the acyl group, an acetyl group, a trifluoroacetyl group, a pivaloyl group, a benzoyl group or a p-phenylbenzoyl group is preferred, and as the cyclic ether group, a tetrahydropyranyl group or a tetrahydrofuranyl group is preferred. As the alkyl group or the aralkyl group which may have a substituent, an alkoxyalkyl group such as a methoxymethyl group, a 1-ethoxyethyl group or a 2-methoxyethoxymethyl group as well as a benzyl group, a methoxybenzyl group or a trityl group may, for example, be mentioned.

As the cyclic acetal or cyclic ketal, methylene acetal, ethylidene acetal or isopropylidene acetal is preferred. As the cyclic orthoester, methoxy methylene acetal or dimethoxy methylene acetal is preferred. As the cyclic silyl ether, a 1,3-(1,1,3,3-tetraisopropyldisiloxanylidene) derivative is preferred, and as the cyclic boronate, phenylboronate is preferred.

The protecting group for a hydroxyl group as mentioned above, can be converted to a hydroxyl group by a conventional method. For example, it can readily be converted to a hydroxyl group by methods disclosed in publications e.g. "Shinjikken Kagaku Koza 14 Syntheses and Reactions of Organic Compounds (I), (II) and (V)", published by Maruzen, and "Protective Groups in Organic Synthesis" written by T. W. Greene, published by J. Wiley & Sons.

The fluorination of a precursory prostaglandin having a hydroxyl group at the 15-position to a fluorine-containing prostaglandin derivative uses a fluorinating agent. The fluorination is preferably carried out in an inert solvent and may be conducted in the presence of a base. The reaction temperature for the fluorination is usually from -150 to +100°C, preferably from -80 to +40°C.

The fluorinating agent used for fluorinating a precursory prostaglandin to a fluorine-containing prostaglandin derivative is not particularly limited, and known or common nucleophilic fluorinating agents may be employed. For example, nucleophilic fluorinating agents disclosed in publications such as "Fusso no Kagaku (Fluorine Chemistry)" written by Tomoya Kitazume, Takashi Ishihara and Takeo Taguchi and published by Kodansha Scientific, may be used. Specifically, dialkylaminosulfur trifluoride derivatives, tetrafluorophenylphophorane, fluoroalkylamine agents such as diethylamine-chlorotrifluoroethene adducts and diethylamine-hexafluoropropene adducts, hydrogen fluoride-amine complexes such as HF-pyridine and HF-triethylamine, silicon tetrafluoride, sulfur tetrafluoride, metal fluorides such as potassium fluoride, cesium fluoride and silver fluoride, and ammonium salts and phosphonium salts such as tetrabutylammonium fluoride, tetraethylammonium fluoride and tetrabutylfluorophosphorane may, for example, be mentioned.

In view of yield and selectivity, dialkylaminosulfur trifluoride derivatives are particularly preferred as the nucleophilic fluorinating agent, and specifically, morpholinosulfur trifluoride, piperidinosulfurtrifluoride, diethylaminosulfur trifluoride, dimethylaminosulfur trifluoride and the like are preferred.

The hydroxyl group of a precursory prostaglandin can be fluorinated directly by using these nucleophilic fluorinating agents, or after conversion of the hydroxyl group into its derivative such as a sulfonate or a silyl ether in order to improve its reactivity or inhibit side reactions. As the sulfonate, a methanesulfonate, a p-toluenesulfonate, a benzenesulfonate or a trifluoromethanesulfonate is preferred, and as the silyl ether, trimethylsilyl ether is preferred.

The amount of a fluorinating agent is usually from 0.5 to 20 parts by weight, preferably from 1 to 5 parts by weight, per part by weight of a precursory prostaglandin as the substrate.

As the inert solvent, a halogen-containing solvent, an etherial solvent, a hydrocarbon solvent, a polar solvent, or a mixture thereof is preferred. An inert solvent is used usually in an amount of from 5 to 1000 parts by weight, preferably from 10 to 100 parts by weight, per part by weight of a prostaglandin.

Preferable halogen-containing solvents are methylene chloride, chloroform, 1,2-dichloroethane, carbon tetrachloride, chlorobenzene and dichloropentafluoropropanes. Preferable etherial solvents are diethyl ether, tetrahydrofuran [THF], 1,4-dioxane, dimethoxyethane, diglyme and t-butyl methyl ether. Preferable hydrocarbon solvents are hexane, toluene, benzene, pentane, xylene and petroleum ether. Preferable polar solvents are dimethyl sulfoxide, hexamethylphosphoramide [HMPA], 1,3-dimethyl-3,4,5,6-tetrahydro-2(1H)-pyrimidinone [DMPU], 1,3-dimethyl-2-imidazolidinone [DMI] and N,N,N',N'-tetramethylethylenediamine [TMEDA] (in the square brackets are abbreviations).

As the base used for the fluorination, amines such as tertiary amines and aromatic amines and salts of alkali metals and alkaline earth metals are preferred. Specifically, triethylamine, diisopropylethylamine, pyridine, 2,6-lutidine, dimethylaminopyridine, sodium carbonate, potassium carbonate, sodium hydrogencarbonate and potassium hydrogencarbonate may be mentioned.

The fluorine-containing prostaglandin derivatives (1) of the present invention can also be prepared by treating a fluorine-containing lactol of the following formula (2) with a phosphorane (3) of the following formula (3). Specifically speaking, the fluorine-containing prostaglandin derivatives (1) of the present invention can be prepared, for example, by treating a fluorine-containing lactol of the formula (2) (hereinafter referred to as a fluorine-containing lactol (2)) obtained by fluorination of an alcohol of the following formula (5) (hereinafter referred to as an alcohol (5)) to a fluorine-containing lactone of the following formula (4) (hereinafter referred to as a fluorine-containing lactone (4)) followed by reduction of the fluorine-containing lactone (4) to the fluorine-containing lactol (2), with a phosphorane of the formula (3) (hereinafter referred to as a phosphorane (3)) derived from a phosphonium salt of the following formula (6) (hereinafter referred to as a phosphonium salt (6)). This procedure will be explained below. In the following formulae (3) and (6), R¹⁰ is a substituted or unsubstituted aryl group, a substituted or unsubstituted alkyl group, a substituted or unsubstituted aralkyl group or a dialkylamino group, and Y is a halogen atom such as a chlorine atom, a bromine atom or an iodine atom.

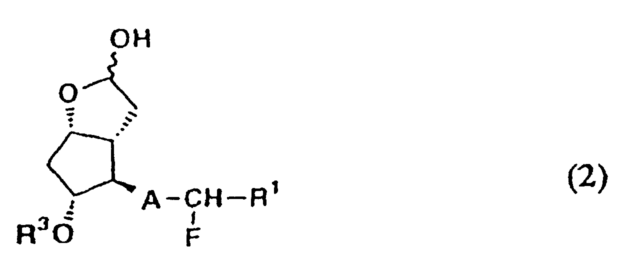

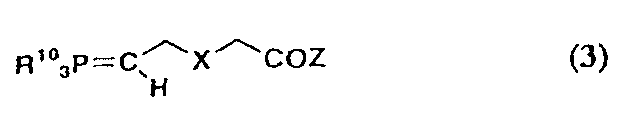

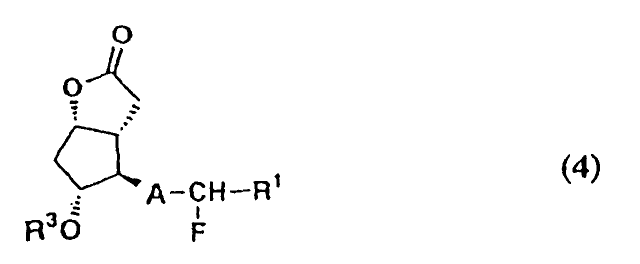

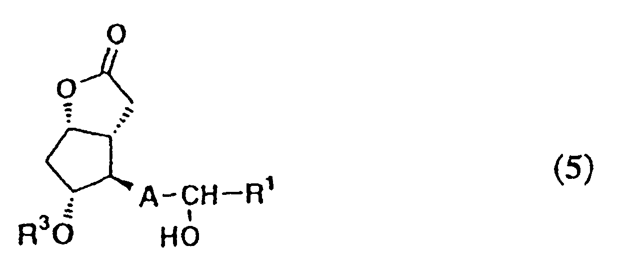

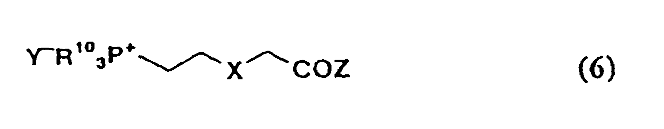

The alcohol (5) is basically a known compound and is prepared by a known method. The alcohol (5) is fluorinated practically by the above-mentioned fluorination method. For the configuration of the hydroxyl group to be fluorinated, protection of a hydroxyl group not to be fluorinated, and fluorination conditions such as the fluorinating agent and solvent to be used and reaction temperature and use of a base, the same as described above applies.

The fluorine-containing lactone (4) obtained by the above-mentioned fluorination is reduced to the fluorine-containing lactol (2). For the reduction, a reducing agent is usually used in an inert solvent. For example, methods disclosed in publications such as "Shinjikken Kagaku Koza 15 Oxidation and reduction (II)" published by Maruzen and "Jikken Kagaku Koza 26 Organic Syntheses VIII, asymmetric synthesis, reduction, sugar and labeled compounds, fourth edition" published by Maruzen may be used.

In the reduction, a reducing agent is used usually in amount of from 0.01 to 50 equivalents, preferably from 5 5 to 20 equivalents, per equivalent of a fluorine-containing lactone (4). The reaction temperature is preferably from -150 to +100°C, particularly preferably from -80 to 0°C.

As reducing agents, diisobutylaluminum hydride [DIBAH], dialkylaluminum alkoxides, lithium aluminum hydride, tributyltin hydride, triphenyltin hydride, triethylsilane, trichlorosilane, dimethylphenylsilane, diphenylsilane, sodium borohydride, sodium trimethoxyborohydride, lithium tri(s-butyl)borohydride, potassium tri(s-butyl)borohydride, lithium triethylborohydride, lithium trisiamylborohydride, potassium trisiamylborohydride, zinc borohydride, calcium borohydride, lithium trialkoxyaluminum hydrides, sodium bis(2-methoxyethoxy)aluminum hydride, diborane, disiamylborane, thexylborane and 9-borabicyclo[3.3.1]nonane may be mentioned. Diisobutylaluminum hydride [DIBAH] and sodium bis(2-methoxyethoxy)aluminum hydride are preferred.

As the inert solvent used for the reduction, an etherial solvent, a hydrocarbon solvent, a polar solvent or a mixture thereof is preferred. Specific examples of the etherial solvent, the hydrocarbon solvent and the polar solvent are the etherial solvents, hydrocarbon solvents and polar solvents specifically described above for the fluorination. Above all, diethyl ether, THF, t-butyl methyl ether, toluene and benzene are particularly preferred. The configuration around the hydroxyl group of the fluorine-containing lactol (2) produced by the reduction is not particularly limited.

As described above, a phosphorane (3) is produced from the corresponding phosphonium salt (6) in an inert solvent in the presence of a base. The resulting phosphorane (3) is not usually isolated and directly used for the Wittig reaction with a fluorine-containing lactol (2). For production of a phosphorane (3) from the corresponding phosphonium salt (6), methods disclosed in publications such as "Shinjikken Kagaku Koza 14 Syntheses and reactions of organic compounds (I)" published by Maruzen, and "Jikken Kagaku Koza 19 Organic Synthesis I, hydrocarbons and halogen compounds, fourth edition" published by Maruzen and the method of Schaaf et al. (J. Med. Chem. 22, 1340 (1979)) may, for example, be employed.

Z in the phosphorane (3) or the phosphonium salt (6) is usually a hydroxyl group (namely, OR⁴ wherein R⁴ is a hydrogen atom) although it may be any of those described above for Z. In such a case, the reaction of the phosphorane (3) with the fluorine-containing lactol (2) gives a fluorine-containing prostaglandin derivative (1) wherein Z is a hydroxyl group. In order to obtain a fluorine-containing prostaglandin derivative (1) wherein Z is not a hydroxyl group, it is preferred to convert Z of a fluorine-containing prostaglandin derivative from a hydroxyl group to a different group. A fluorine-containing prostaglandin derivative (1) wherein Z is not a hydroxyl group can also be prepared from a phosphorane (3) or its precursor having a group other than a hydroxyl group as Z.

Conversion of a phosphonium salt (6) wherein Z is -NHCOR⁵ or-NHSO₂R⁶ to a phosphorane (3) is sometimes accompanied by replacement of the hydrogen atom bonded to the nitrogen atom in -NHCOR⁵ or -NHSO₂R⁶ by a metal ion. Consequently, in such a case, the product of the Wittig reaction of the resulting phosphorane with a fluorine-containing lactol (2) also has a metal ion at the corresponding site. The metal ion is attributed to the metal ion (specifically, an alkali metal ion or an alkaline earth metal ion) in the base used for conversion of a phosphonium salt (6) into a phosphorane (3). The metal ion can, if necessary, be replaced by a hydrogen atom through hydrolysis or other processes.

A phosphorane (3) is used usually in an amount of from 0.1 to 20 equivalents, preferably from 1 to 10 equivalents, per equivalent of a fluorine-containing lactol (2). The reaction of a fluorine-containing lactol (2) with a phosphorane (3) is classified as the so-called Wittig reaction. Ordinary conditions for the Wittig reaction are applicable to the reaction of a fluorine-containing lactol (2) with a phosphorane (3) according to the present invention. In particular, it is preferred to carry out the reaction under basic conditions in an inert solvent. The reaction temperature is usually from -150 to +200°C, preferably from -80 to +100°C.

A base is used usually in an amount of from 0.1 to 20 equivalents, preferably from 1 to 10 equivalents, per equivalent of a fluorine-containing lactol (2). A base of the proper kind should be used in view of the acidity of the hydrogen atom bonded to the carbon atom at the α-position based on the phosphorus atom of a phosphonium salt (6) as the precursor of a phosphorane (3) and the stability of the resulting phosphorane (3). Such a base can be selected, for example, from the following bases.

Sodium hydroxide, potassium hydroxide, sodium carbonate, potassium carbonate, sodium methoxide, sodium ethoxide, triethylamine, diisopropylethylamine, pyridine, N-methylmorpholine, diazabicyclononene, diazabicycloundecene, potassium t-butoxide, lithium amide, sodium amide, potassium amide, lithium diisopropylamide, lithium diethylamide, lithium dicyclohexylamide, lithium isopropylcyclohexylamide, lithium 2,2,6,6-tetramethylpiperidine, lithium hexamethyldisilazide, sodium diethylamide, sodium hexamethyldisilazide, potassium 3-aminopropylamide, potassium hexamethyldisilazide, lithium hydride, sodium hydride, potassium hydride, sodium dimsyl, n-butyllithium, s-butyllithium, t-butyllithium, methyllithium, phenyllithium, lithium naphthalenide, lithium biphenylide and tritylsodium.

Among these bases, potassium carbonate, potassium t-butoxide, lithium amide, sodium amide, potassium amide, lithium diisopropylamide, lithium diethylamide, lithium dicyclohexylamide, lithium isopropylcyclohexylamide, lithium 2,2,6,6-tetramethylpiperidine, lithium hexamethyldisilazide, sodium diethylamide, sodium hexamethyldisilazide, potassium 3-aminopropylamide, potassium hexamethyldisilazide and sodium dimsyl are preferred. Potassium t-butoxide, sodium hexamethyldisilazide, potassium hexamethyldisilazide and sodium dimsyl are particularly preferred.

As the inert solvent, an etherial solvent, a hydrocarbon solvent, a polar solvent, an aqueous solvent, an alcoholic solvent or a solvent mixture thereof is preferred. An inert solvent is used usually in an amount of from 5 to 1000 parts by weight, preferably from 10 to 100 parts by weight, per part by weight of a fluorine-containing lactol (2). As specific examples of the etherial solvent, the hydrocarbon solvent and the polar solvent, the etherial solvents, the hydrocarbon solvents and the polar solvents specifically described above for the fluorination are preferred. As the aqueous solvent, water or a solvent mixture of water with an alcoholic solvent is preferred. As the alcoholic solvent, methanol, ethanol, t-butanol and t-amyl alcohol are preferred. Particularly preferred solvents are diethyl ether, THF, 1,2-dimethoxyethane, t-butyl methyl ether, toluene and benzene.

Z of the resulting fluorine-containing prostaglandin derivative (1) can be converted into a different kind of Z, if necessary. For example, a fluorine-containing prostaglandin derivative wherein Z is a hydroxyl group can optionally be converted into an ester, a salt of a carboxylic acid, an acyl amide, sulfonamide or a thioester by a conventional method.

For esterification of Z, ordinary methods such as methods disclosed in publications such as "Shinjikken Kagaku Koza 14 Syntheses and reactions of organic compounds (II)" published by Maruzen may be used. Esterification by condensation with an alcohol or a phenol, esterification with an O-alkylating agent, esterification by use of an alkene or an alkyne, esterification with a dialkyl sulfate or a halogenated hydrocarbon may be mentioned.

For conversion into an acylamide or a sulfonamide, the method of Tithereley et al. (J. Chem. Soc. 85, 1673 (1904)), the method of Lynch et al. (Can. J. Chem. 50, 2143 (1972)), the method of Davidson et al. (J. Am. Chem. Soc. 80, 376 (1958)) and the like can be used. Alternatively, conversion of a carboxylic acid into an acid halide or a reactive ester followed by condensation with an amide or a sulfonamide or reaction of a carboxylic acid with an amine to produce an amide followed by acylation or sulfonylation may be employed.

For conversion of Z into a thioester, methods described in publications such as "Shinjikken Kagaku Koza 14 Syntheses and reactions of organic compounds (III)" published by Maruzen and "Protective Groups in Organic Syntheses" written by T.W. Greene and published by J. Wiley & Sons may be employed. For example, a process wherein a carboxylic acid is converted into an acid halide or a reactive ester and then reacted with a thiol may be employed.

Specific examples of the compound of the formula (1) are given below, and, however, the compound is not limited to these specific examples.
16-(3,5-Dichlorophenoxy)-15-deoxy-15-fluoro-17,18,19,20-tetranorprostaglandin F_{2α} methyl ester,
16-(3,5-dichlorophenoxy)-15-deoxy-15-fluoro-17,18,19,20-tetranorprostaglandin F_{2α} ethyl ester,
16-(3,5-dichlorophenoxy)-15-deoxy-15-fluoro-17,18,19,20-tetranorprostaglandin F_{2α} isopropyl ester,
16-(3,4-dichlorophenoxy)-15-deoxy-15-fluoro-17,18,19,20-tetranorprostaglandin F_{2α} methyl ester,
16-(3,4-dichlorophenoxy)-15-deoxy-15-fluoro-17,18,19,20-tetranorprostaglandin F_{2α} ethyl ester,
16-(3,4-dichlorophenoxy)-15-deoxy-15-fluoro-17,18,19,20-tetranorprostaglandin F_{2α} isopropyl ester,
16-(3-chlorophenoxy)-15-deoxy-15-fluoro-17,18,19,20-tetranorprostaglandin F_{2α} methyl ester,
16-(3-chlorophenoxy)-15-deoxy-15-fluoro-17,18,19,20-tetranorprostaglandin F_{2α} ethyl ester,
16-(3-chlorophenoxy)-15-deoxy-15-fluoro-17,18,19,20-tetranorprostaglandin F_{2α} isopropyl ester,
16-(3,5-difluorophenoxy)-15-deoxy-15-fluoro-17,18,19,20-tetranorprostaglandin F_{2α} methyl ester,
16-(3,5-difluorophenoxy)-15-deoxy-15-fluoro-17,18,19,20-tetranorprostaglandin F_{2α} ethyl ester,
16-(3,5-difluorophenoxy)-15-deoxy-15-fluoro-17,18,19,20-tetranorprostaglandin F_{2α} isopropyl ester,
16-(3,4-difluorophenoxy)-15-deoxy-15-fluoro-17,18,19,20-tetranorprostaglandin F_{2α} methyl ester,
16-(3,4-difluorophenoxy)-15-deoxy-15-fluoro-17,18,19,20-tetranorprostaglandin F_{2α} ethyl ester,
16-(3,4-difluorophenoxy)-15-deoxy-15-fluoro-17,18,19,20-tetranorprostaglandin F_{2α} isopropyl ester,
16-(3-fluorophenoxy)-15-deoxy-15-fluoro-17,18,19,20-tetranorprostaglandin F_{2α} methyl ester,
16-(3-fluorophenoxy)-15-deoxy-15-fluoro-17,18,19,20-tetranorprostaglandin F_{2α} ethyl ester,
16-(3-fluorophenoxy)-15-deoxy-15-fluoro-17,18,19,20-tetranorprostaglandin F_{2α} isopropyl ester,
16-[3,5-bis(trifluoromethyl)phenoxy]-15-deoxy-15-fluoro-17,18,19,20-tetranorprostaglandin F_{2α} methyl ester,
16-[3,5-bis(trifluoromethyl)phenoxy]-15-deoxy-15-fluoro-17,18,19,20-tetranorprostaglandin F_{2α} ethyl ester,
16-[3,5-bis(trifluoromethyl)phenoxy]-15-deoxy-15-fluoro-17,18,19,20-tetranorprostaglandin F_{2α} isopropyl ester,
16-[3,4-bis(trifluoromethyl)phenoxy]-15-deoxy-15-fluoro-17,18,19,20-tetranorprostaglandin F_{2α} methyl ester,
16-[3,4-bis(trifluoromethyl)phenoxy]-15-deoxy-15-fluoro-17,18,19,20-tetranorprostaglandin F_{2α} ethyl ester,
16-[3,4-bis(trifluoromethyl)phenoxy]-15-deoxy-15-fluoro-17,18,19,20-tetranorprostaglandin F_{2α} isopropyl ester,
16-[3-(trifluoromethyl)phenoxy]-15-deoxy-15-fluoro-17,18,19,20-tetranorprostaglandin.F_{2α} methyl ester,
16-[3-(trifluoromethyl)phenoxy]-15-deoxy-15-fluoro-17,18,19,20-tetranorprostaglandin F_{2α} ethyl ester,
16-[3-(trifluoromethyl)phenoxy]-15-deoxy-15-fluoro-17,18,19,20-tetranorprostaglandin F_{2α} isopropyl ester,
16-phenoxy-15-deoxy-15-fluoro-17,18,19,20-tetranorprostaglandin F_{2α} methyl ester,
16-phenoxy-15-deoxy-15-fluoro-17,18,19,20-tetranorprostaglandin F_{2α} ethyl ester,
16-phenoxy-15-deoxy-15-fluoro-17,18,19,20-tetranorprostaglandin F_{2α} isopropyl ester,
16-(3-methylphenyl)-15-deoxy-15-fluoro-17,18,19,20-tetranorprostaglandin F_{2α} methyl ester,
16-(3-methylphenyl)-15-deoxy-15-fluoro-17,18,19,20-tetranorprostaglandin F_{2α} ethyl ester,
16-(3-methylphenyl)-15-deoxy-15-fluoro-17,18,19,20-tetranorprostaglandin F_{2α} isopropyl ester,
16-[3-(trifluoromethyl)phenyl]-15-deoxy-15-fluoro-17,18,19,20-tetranorprostaglandin F_{2α} methyl ester,
16-[3-(trifluoromethyl)phenyl]-15-deoxy-15-fluoro-17,18,19,20-tetranorprostaglandin F_{2α} ethyl ester,
16-[3-(trifluoromethyl)phenyl]-15-deoxy-15-fluoro-17,18,19,20-tetranorprostaglandin F_{2α} isopropyl ester,
16-phenyl-15-deoxy-15-fluoro-17,18,19,20-tetranorprostaglandin F_{2α} methyl ester,
16-phenyl-15-deoxy-15-fluoro-17,18,19,20-tetranorprostaglandin F_{2α} ethyl ester,
16-phenyl-15-deoxy-15-fluoro-17,18,19,20-tetranorprostaglandin F_{2α} isopropyl ester,
17-phenyl-15-deoxy-15-fluoro-18,19,20-trinorprostaglandin F_{2α} methyl ester,
17-phenyl-15-deoxy-15-fluoro-18,19,20-trinorprostaglandin F_{2α} ethyl ester,
17-phenyl-15-deoxy-15-fluoro-18,19,20-trinorprostaglandin F_{2α} isopropyl ester,
15-deoxy-15-fluoro-13,14-dihydroprostaglandin F_{2α} methyl ester,
15-deoxy-15-fluoro-13,14-dihydroprostaglandin F_{2α} ethyl ester,
15-deoxy-15-fluoro-13,14-dihydroprostaglandin F_{2α} isopropyl ester,
16-(3,5-dichlorophenoxy)-15-deoxy-15-fluoro-13,14-dihydro-17,18,19,20-tetranorprostaglandin F_{2α} methyl ester,
16-(3,5-dichlorophenoxy)-15-deoxy-15-fluoro-13,14-dihydro-17,18,19,20-tetranorprostaglandin F_{2α} ethyl ester,
16-(3,5-dichlorophenoxy)-15-deoxy-15-fluoro-13,14-dihydro-17,18,19,20-tetranorprostaglandin F_{2α} isopropyl ester,
16-(3,4-dichlorophenoxy)-15-deoxy-15-fluoro-13,14-dihydro-17,18,19,20-tetranorprostaglandin F_{2α} methyl ester,
16-(3,4-dichlorophenoxy)-15-deoxy-15-fluoro-13,14-dihydro-17,18,19,20-tetranorprostaglandin F_{2α} ethyl ester,
16-(3,4-dichlorophenoxy)-15-deoxy-15-fluoro-13,14-dihydro-17,18,19,20-tetranorprostaglandin F_{2α} isopropyl ester,
16-(3-chlorophenoxy)-15-deoxy-15-fluoro-13,14-dihydro-17,18,19,20-tetranorprostaglandin F_{2α} methyl ester,
16-(3-chlorophenoxy)-15-deoxy-15-fluoro-13,14-dihydro-17,18,19,20-tetranorprostaglandin F_{2α} ethyl ester,
16-(3-chiorophenoxy)-15-deoxy-15-fluoro-13,14-dihydro-17,18,19,20-tetranorprostaglandin F_{2α} isopropyl ester,
16-phenoxy-15-deoxy-15-fluoro-13,14-dihydro-17,18,19,20-tetranorprostaglandin F_{2α} methyl ester,
16-phenoxy-15-deoxy-15-fluoro-13,14-dihydro-17,18,19,20-tetranorprostaglandin F_{2α} ethyl ester,
16-phenoxy-15-deoxy-15-fluoro-13,14-dihydro-17,18,19,20-tetranorprostaglandin F_{2α} isopropyl ester,
17-phenyl-15-deoxy-15-fluoro-13,14-dihydro-18,19,20-trinorprostaglandin F_{2α} methyl ester,
17-phenyl-15-deoxy-15-fluoro-13,14-dihydro-18,19,20-trinorprostaglandin F_{2α} ethyl ester,
17-phenyl-15-deoxy-15-fluoro-13,14-dihydro-18,19,20-trinorprostaglandin F_{2α} isopropyl ester,
16-(3,5-dichlorophenoxy)-15-deoxy-15-fluoro-3-oxa-17,18,19,20-tetranorprostaglandin F_{2α} methyl ester,
16-(3,5-dichlorophenoxy)-15-deoxy-15-fluoro-3-oxa-17,18,19,20-tetranorprostaglandin F_{2α} ethyl ester,
16-(3,5-dichlorophenoxy)-15-deoxy-15-fluoro-3-oxa-17,18,19,20-tetranorprostaglandin F_{2α} isopropyl ester,
16-(3,4-dichlorophenoxy)-15-deoxy-15-fluoro-3-oxa-17,18,19,20-tetranorprostaglandin F_{2α} methyl ester,
16-(3,4-dichlorophenoxy)-15-deoxy-15-fluoro-3-oxa-17,18,19,20-tetranorprostaglandin F_{2α} ethyl ester,
16-(3,4-dichlorophenoxy)-15-deoxy-15-fluoro-3-oxa-17,18,19,20-tetranorprostaglandin F_{2α} isopropyl ester,
16-(3-chlorophenoxy)-15-deoxy-15-fluoro-3-oxa-17,18,19,20-tetranorprostaglandin F_{2α} methyl ester,
16-(3-chlorophenoxy)-15-deoxy-15-fluoro-3-oxa-17,18,19,20-tetranorprostaglandin F_{2α} ethyl ester,
16-(3-chlorophenoxy)-15-deoxy-15-fluoro-3-oxa-17,18,19,20-tetranorprostaglandin F_{2α} isopropyl ester,
15-deoxy-15-fluoro-9-pivaloylprostaglandin F_{2α} methyl ester,
15-deoxy-15-fluoro-9-pivaloylprostaglandin F_{2α} ethyl ester,
15-deoxy-15-fluoro-9-pivaloylprostaglandin F_{2α} isopropyl ester,
16-(3,5-dichlorophenoxy)-15-deoxy-15-fluoro-9-pivaloyl-17,18,19,20-tetranorprostaglandin F_{2α} methyl ester,
16-(3,5-dichlorophenoxy)-15-deoxy-15-fluoro-9-pivaloyl-17,18,19,20-tetranorprostaglandin F_{2α} ethyl ester,
16-(3,5-dichlorophenoxy)-15-deoxy-15-fluoro-9-pivaloyl-17,18,19,20-tetranorprostaglandin F_{2α} isopropyl ester,
16-(3,4-dichlorophenoxy)-15-deoxy-15-fluoro-9-pivaloyl-17,18,19,20-tetranorprostaglandin F_{2α} methyl ester,
16-(3,4-dichlorophenoxy)-15-deoxy-15-fluoro-9-pivaloyl-17,18,19,20-tetranorprostaglandin F_{2α} ethyl ester,
16-(3,4-dichlorophenoxy)-15-deoxy-15-fluoro-9-pivaloyl-17,18,19,20-tetranorprostaglandin F_{2α} isopropyl ester,
16-(3-chlorophenoxy)-15-deoxy-15-fluoro-9-pivaloyl-17,18,19,20-tetranorprostaglandin F_{2α} methyl ester,
16-(3-chlorophenoxy)-15-deoxy-15-fluoro-9-pivaloyl-17,18,19,20-tetranorprostaglandin F_{2α} ethyl ester,
16-(3-chlorophenoxy)-15-deoxy-15-fluoro-9-pivaloyl-17,18,19,20-tetranorprostaglandin F_{2α} isopropyl ester,
16-(3,5-difluorophenoxy)-15-deoxy-15-fluoro-9-pivaloyl-17,18,19,20-tetranorprostaglandin F_{2α} methyl ester,
16-(3,5-difluorophenoxy)-15-deoxy-15-fluoro-9-pivaloyl-17,18,19,20-tetranorprostaglandin F_{2α} ethyl ester,
16-(3,5-difluorophenoxy)-15-deoxy-15-fluoro-9-pivaloyl-17,18,19,20-tetranorprostaglandin F_{2α} isopropyl ester,
16-(3,4-difluorophenoxy)-15-deoxy-15-fluoro-9-pivaloyl-17,18,19,20-tetranorprostaglandin F_{2α} methyl ester,
16-(3,4-difluorophenoxy)-15-deoxy-15-fluoro-9-pivaloyl-17,18,19,20-tetranorprostaglandin F_{2α} ethyl ester,
16-(3,4-difluorophenoxy)-15-deoxy-15-fluoro-9-pivaloyl-17,18,19,20-tetranorprostaglandin F_{2α} isopropyl ester,
16-(3-fluorophenoxy)-15-deoxy-15-fluoro-9-pivaloyl-17,18,19,20-tetranorprostaglandin F_{2α} methyl ester,
16-(3-fluorophenoxy)-15-deoxy-15-fluoro-9-pivaloyl-17,18,19,20-tetranorprostaglandin F_{2α} ethyl ester,
16-(3-fluorophenoxy)-15-deoxy-15-fluoro-9-pivaloyl-17,18,19,20-tetranorprostaglandin F_{2α} isopropyl ester,
16-[3,5-bis(trifluoromethyl)phenoxy]-15-deoxy-15-fluoro-9-pivaloyl-17,18,19,20-tetranorprostaglandin F_{2α} methyl ester,
16-[3,5-bis(trifluoromethyl)phenoxy]-15-deoxy-15-fluoro-9-pivaloyl-17,18,19,20-letranorprostagiandin F_{2α} ethyl ester,
16-[3,5-bis(trifluoromethyl)phenoxy]-15-deoxy-15-fluoro-9-pivaloyl-17,18,19,20-tetranorprostaglandin F_{2α} isopropyl ester,
16-[3,4-bis(trifluoromethyl)phenoxy]-15-deoxy-15-fluoro-9-pivaloyl-17,18,19,20-tetranorprostaglandin F_{2α} methyl ester,
16-[3,4-bis(trifluoromethyl)phenoxy]-15-deoxy-15-fluoro-9-pivaloyl-17,18,19,20-tetranorprostaglandin F_{2α} ethyl ester,
16-[3,4-bis(trifluoromethyl)phenoxy]-15-deoxy-15-fluoro-9-pivaloyl-17,18,19,20-tetranorprostaglandin F_{2α} isopropyl ester,
16-[3-(trifluoromethyl)phenoxy] -15-deoxy-15-fluoro-9-pivaloyl-17,18,19,20-tetranorprostaglandin F_{2α} methyl ester,
16-[3-(trifluoromethyl)phenoxy)-15-deoxy-15-fluoro-9-pivaloyl-17,18,19,20-tetranorprostaglandin F_{2α} ethyl ester,
16-[3-(trifluoromethyl)phenoxy]-15-deoxy-15-fluoro-9-pivaloyl-17,18,19,20-tetranorprostaglandin F_{2α} isopropyl ester,
16-(3-methylphenyl)-15-deoxy-15-fluoro-9-pivaloyl-17,18,19,20-tetranorprostaglandin F_{2α} methyl ester,
16-(3-methylphenyl)-15-deoxy-15-fluoro-9-pivaloyl-17,18,19,20-tetranorprostaglandin F_{2α} ethyl ester,
16-(3-methylphenyl)-15-deoxy-15-fluoro-9-pivaloyl-17,18,19,20-tetranorprostaglandin F_{2α} isopropyl ester,
16-[3-(trifluoromethyl)phenyl]-15-deoxy-15-fluoro-9-pivaloyl-17,18,19,20-tetranorprostaglandin F_{2α} methyl ester,
16-[3-(trifluoromethyl)phenyl]-15-deoxy-15-fluoro-9-pivaloyl-17,18,19,20-tetranorprostaglandin F_{2α} ethyl ester,
16-[3-(trifluoromethyl)phenyl]-15-deoxy-15-fluoro-9-pivaloyl-17,18,19,20-tetranorprostaglandin F_{2α} isopropyl ester,
16-phenyl-15-deoxy-15-fluoro-9-pivaloyl-17,18,19,20-tetranorprostaglandin F_{2α} methyl ester,
16-phenyl-15-deoxy-15-fluoro-9-pivaloyl-17,18,19,20-tetranorprostaglandin F_{2α} ethyl ester,
16-phenyl-15-deoxy-15-fluoro-9-pivaloyl-17,18,19,20-tetranorprostaglandin F_{2α} isopropyl ester,
17-phenyl-15-deoxy-15-fluoro-9-pivaloyl-18,19,20-trinorprostaglandin F_{2α} methyl ester,
17-phenyl-15-deoxy-15-fluoro-9-pivaloyl-18,19,20-trinorprostaglandin F_{2α} ethyl ester,
17-phenyl-15-deoxy-15-fluoro-9-pivaloyl-18,19,20-trinorprostaglandin F_{2α} isopropyl ester,
15-deoxy-15-fluoro-11-pivaloylprostaglandin F_{2α} methyl ester,
15-deoxy-15-fluoro-11-pivaloylprostaglandin F_{2α} ethyl ester,
15-deoxy-15-fluoro-11-pivaloylprostaglandin F_{2α} isopropyl ester,
16-(3,5-dichlorophenoxy)-15-deoxy-15-fluoro-11-pivaloyl-17,18,19,20-tetranorprostaglandin F_{2α} methyl ester,
16-(3,5-dichlorophenoxy)-15-deoxy-15-fluoro-11-pivaloyl-17,18,19,20-tetranorprostaglandin F_{2α} ethyl ester,
16-(3,5-dichlorophenoxy)-15-deoxy-15-fluoro-11-pivaloyl-17,18,19,20-tetranorprostaglandin F_{2α} isopropyl ester,
16-(3,4-dichlorophenoxy)-15-deoxy-15-fluoro-11-pivaloyl-17,18,19,20-tetranorprostaglandin F_{2α} methyl ester,
16-(3,4-dichlorophenoxy)-15-deoxy-15-fluoro-11-pivaloyl-17,18,19,20-tetranorprostaglandin F_{2α} ethyl ester,
16-(3,4-dichlorophenoxy)-15-deoxy-15-fluoro-11-pivaloyl-17,18,19,20-tetranorprostaglandin F_{2α} isopropyl ester,
16-(3-chlorophenoxy)-15-deoxy-15-fluoro-11-pivaloyl-17,18,19,20-tetranorprostaglandin F_{2α} methyl ester,
16-(3-chlorophenoxy)-15-deoxy-15-fluoro-11-pivaloyl-17,18,19,20-tetranorprostaglandin F_{2α} ethyl ester,
16-(3-chlorophenoxy)-15-deoxy-15-fluoro-11-pivaloyl-17,18,19,20-tetranorprostaglandin F_{2α} isopropyl ester,
16-(3,5-difluorophenoxy)-15-deoxy-15-fluoro-11-pivaloyl-17,18,19,20-tetranorprostaglandin F_{2α} methyl ester,
16-(3,5-difluorophenoxy)-15-deoxy-15-fluoro-11-pivaloyl-17,18,19,20-tetranorprostaglandin F_{2α} ethyl ester,
16-(3,5-difluorophenoxy)-15-deoxy-15-fluoro-11-pivaloyl-17,18,19,20-tetranorprostaglandin F_{2α} isopropyl ester,
16-(3,4-difluorophenoxy)-15-deoxy-15-fluoro-11-pivaloyl-17,18,19,20-tetranorprostaglandin F_{2α} methyl ester,
16-(3,4-difluorophenoxy)-15-deoxy-15-fluoro-11-pivaloyl-17,18,19,20-tetranorprostaglandin F_{2α} ethyl ester,
16-(3,4-difluorophenoxy)-15-deoxy-15-fluoro-11-pivaloyl-17,18,19,20-tetranorprostaglandin F_{2α} isopropyl ester,
16-(3-fluorophenoxy)-15-deoxy-15-fluoro-11-pivaloyl-17,18,19,20-tetranorprostaglandin F_{2α} methyl ester,
16-(3-fluorophenoxy)-15-deoxy-15-fluoro-11-pivaloyl-17,18,19,20-tetranorprostaglandin F_{2α} ethyl ester,
16-(3-fluorophenoxy)-15-deoxy-15-fluoro-11-pivaloyl-17,18,19,20-tetranorprostaglandin F_{2α} isopropyl ester,
16-[3,5-bis(trifluoromethyl)phenoxy]-15-deoxy-15-fluoro-11-pivaloyl-17,18,19,20-tetranorprostaglandin F_{2α} methyl ester,
16-[3,5-bis(trifluoromethyl)phenoxy]-15-deoxy-15-fluoro-11-pivaloyl-17,18,19,20-tetranorprostaglandin F_{2α} ethyl ester,
16-[3,5-bis(trifluoromethyl)phenoxy]-15-deoxy-15-fluoro-11-pivaloyl-17,18,19,20-tetranorprostaglandin F_{2α} isopropyl ester,
16-[3,4-bis(trifluoromethyl)phenoxy]-15-deoxy-15-fluoro-11-pivaloyl-17,18,19,20-tetranorprostaglandin F_{2α} methyl ester,
16-[3,4-bis(trifluoromethyl)phenoxy]-15-deoxy-15-fluoro-11-pivaloyl-17,18,19,20-tetranorprostaglandin F_{2α} ethyl ester,
16-[3,4-bis(trifluoromethyl)phenoxy]-15-deoxy-15-fluoro-11-pivaloyl-17,18,19,20-tetranorprostaglandin F_{2α} isopropyl ester,
16-[3-(trifluoromethyl)phenoxy]-15-deoxy-15-fluoro-11-pivaloyl-17,18,19,20-tetranorprostaglandin F_{2α} methyl ester,
16-[3-(trifluoromethyl)phenoxy]-15-deoxy-15-fluoro-11-pivaloyl-17,18,19,20-tetranorprostaglandin F_{2α} ethyl ester,
16-[3-(trifluoromethyl)phenoxy]-15-deoxy-15-fluoro-11-pivaloyl-17,18,19,20-tetranorprostaglandin F_{2α} isopropyl ester,
16-(3-methylphenyl)-15-deoxy-15-fluoro-11-pivaloyl-17,18,19,20-tetranorprostaglandin F_{2α} methyl ester,
16-(3-methylphenyl)-15-deoxy-15-fluoro-11-pivaloyl-17,18,19,20-tetranorprostaglandin F_{2α} ethyl ester,
16-(3-methylphenyl)-15-deoxy-15-fluoro-11-pivaloyl-17,18,19,20-tetranorprostaglandin F_{2α} isopropyl ester,
16-[3-(trifluoromethyl)phenyl]-15-deoxy-15-fluoro-11-pivaloyl-17,18,19,20-tetranorprostaglandin F_{2α} methyl ester,
16-[3-(trifluoromethyl)phenyl]-15-deoxy-15-fluoro-11-pivaloyl-17,18,19,20-tetranorprostaglandin F_{2α} ethyl ester,
16-[3-(trifluoromethyl)phenyl]-15-deoxy-15-fluoro-11-pivaloyl-17,18,19,20-tetranorprostaglandin F_{2α} isopropyl ester,
16-phenyl-15-deoxy-15-fluoro-11-pivaloyl-17,18,19,20-tetranorprostaglandin F_{2α} methyl ester,
16-phenyl-15-deoxy-15-fluoro-11-pivaloyl-17,18,19,20-tetranorprostaglandin F_{2α} ethyl ester,
16-phenyl-15-deoxy-15-fluoro-11-pivaloyl-17,18,19,20-tetranorprostaglandin F_{2α} isopropyl ester,
17-phenyl-15-deoxy-15-fluoro-11-pivaloyl-18,19,20-trinorprostaglandin F_{2α} methyl ester,
17-phenyl-15-deoxy-15-fluoro-11-pivaloyl-18,19,20-trinorprostaglandin F_{2α} ethyl ester,
17-phenyl-15-deoxy-15-fluoro-11-pivaloyl-18,19,20-trinorprostaglandin F_{2α} isopropyl ester,
15-deoxy-15-fluoroprostaglandin F_{2α} 1,9-lactone,
15-deoxy-15-fluoroprostaglandin F_{2α} 1,11-lactone,
16-(3,5-dichlorophenoxy)-15-deoxy-15-fluoro-17,18,19,20-tetranorprostaglandin F_{2α} 1,9-lactone,
16-(3,5-dichlorophenoxy)-15-deoxy-15-fluoro-17,18,19,20-tetranorprostaglandin F_{2α} 1,11-lactone,
16-(3,4-dichlorophenoxy)-15-deoxy-15-fluoro-17,18,19,20-tetranorprostaglandin F_{2α} 1,9-lactone,
16-(3,4-dichlorophenoxy)-15-deoxy-15-fluoro-17,18,19,20-tetranorprostaglandin F_{2α} 1,11-lactone,
16-(3-chlorophenoxy)-15-deoxy-15-fluoro-17,18,19,20-tetranorprostaglandin F_{2α} 1,9-lactone,
16- (3-chlorophenoxy)-15-deoxy-15-fluoro-17,18,19,20-tetranorprostaglandin F_{2α} 1,11-lactone,
16-(3,5-difluorophenoxy)-15-deoxy-15-fluoro-17,18,19,20-tetranorprostaglandin F_{2α} 1,9-lactone,
16-(3,5-difluorophenoxy)-15-deoxy-15-fluoro-17,18,19,20-tetranorprostaglandin F_{2α} 1,11-lactone,
16-(3,4-difluorophenoxy)-15-deoxy-15-fluoro-17,18,19,20-tetranorprostaglandin F_{2α} 1,9-lactone,
16-(3,4-difluorophenoxy)-15-deoxy-15-fluoro-17,18,19,20-tetranorprostaglandin F_{2α} 1,11-lactone,
16-(3-fluorophenoxy)-15-deoxy-15-fluoro-17,18,19,20-tetranorprostaglandin F_{2α} 1,9-lactone,
16-(3-fluorophenoxy)-15-deoxy-15-fluoro-17,18,19,20-tetranorprostaglandin F_{2α} 1,11-lactone,
16-[3,5-bis(trifluoromethyl)phenoxy]-15-deoxy-15-fluoro-17,18,19,20-tetranorprostaglandin F_{2α} 1,9-lactone,
16-[3,5-bis(trifluoromethyl)phenoxy]-15-deoxy-15-fluoro-17,18,19,20-tetranorprosiaglandin F_{2α} 1,11-lactone,
16-[3,4-bis(trifluoromethyl)phenoxy]-15-deoxy-15-fluoro-17,18,19,20-tetranorprostaglandin F_{2α} 1,9-lactone,
16- [3,4-bis(trifluoromethyl)phenoxy]-15-deoxy-15-fluoro-17,18,19,20-tetranorprostaglandin F_{2α} 1,11-lactone,
16-[3-(trifluoromethyl)phenoxy]-15-deoxy-15-fluoro-17,18,19,20-tetranorprostaglandin F_{2α} 1,9-lactone,
16-[3-(trifluoromethyl)phenoxy]-15-deoxy-15-fluoro-17,18,19,20-tetranorprostaglandin F_{2α} 1,11-lactone,
16-[3-(trifluoromethyl)phenyl]-15-deoxy-15-fluoro-17,18,19,20-tetranorprostaglandin F_{2α} 1,9-lactone,
16-[3-(trifluoromethyl)phenyl]-15-deoxy-15-fluoro-17,18,19,20-tetranorprostaglandin F_{2α} 1,11-lactone,
16-phenyl-15-deoxy-15-fluoro-17,18,19,20-tetranorprostaglandin F_{2α} 1,9-lactone,
16-phenyl-15-deoxy-15-fluoro-17,18,19,20-tetranorprostaglandin F_{2α} 1,11-lactone,
17-phenyl-15-deoxy-15-fluoro-18,19,20-trinorprostaglandin F_{2α} 1,9-lactone,
17-phenyl-15-deoxy-15-fluoro-18,19,20-trinorprostaglandin F_{2α} 1,11-lactone,
16-(3,5-dichlorophenoxy)-15-deoxy-15-fluoro-17,18,19,20-tetranorprostaglandin F_{2α},
16-(3,4-dichlorophenoxy)-15-deoxy-15-fluoro-17,18,19,20-tetranorprostaglandin F_{2α},
16-(3-chlorophenoxy)-15-deoxy-15-fluoro-17,18,19,20-tetranorprostaglandin F_{2α},
16-(3,5-difluorophenoxy)-15-deoxy-15-fluoro-17,18,19,20-tetranorprostaglandin F_{2α},
16-(3,4-difluorophenoxy)-15-deoxy-15-fluoro-17,18,19,20-tetranorprostaglandin F_{2α},
16-(3-fluorophenoxy)-15-deoxy-15-fluoro-17,18,19,20-tetranorprostaglandin F_{2α},
16-[3,5-bis(trifluoromethyl)phenoxy]-15-deoxy-15-fluoro-17,18,19,20-tetranorprostaglandin F_{2α},
16-[3,4-bis(trifluoromethyl)phenoxy]-15-deoxy-15-fluoro-17,18,19,20-tetranorprostaglandin F_{2α},
16-[3-(trifluoromethyl)phenoxy]-15-deoxy-15-fluoro-17,18,19,20-tetranorprostaglandin F_{2α},
16-(3-methylphenyl)-15-deoxy-15-fluoro-17,18,19,20-tetranorprostaglandin F_{2α},
16-[3-(trifluoromethyl)phenyl]-15-deoxy-15-fluoro-17,18,19,20-tetranorprostaglandin F_{2α},
16-phenyl-15-deoxy-15-fluoro-17,18,19,20-tetranorprostaglandin F_{2α},
17-phenyl-15-deoxy-15-fluoro-18,19,20-trinorprostaglandin F_{2α},
15-deoxy-15-fluoro-13,14-dihydroprostaglandin F_{2α},
16-(3,5-dichlorophenoxy)-15-deoxy-15-fluoro-13,14-dihydro-17,18,19,20-tetranorprostaglandin F_{2α},
16-(3,4-dichlorophenoxy)-15-deoxy-15-fluoro-13,14-dihydro-17,18,19,20-tetranorprostaglandin F_{2α},
16-(3-chlorophenoxy)-15-deoxy-15-fluoro-13,14-dihydro-17,18,19,20-tetranorprostaglandin F_{2α},
17-phenyl-15-deoxy-15-fluoro-13,14-dihydro-18,19,20-trinorprostaglandin F_{2α},
16-(3,5-dichlorophenoxy)-15-deoxy-15-fluoro-3-oxa-17,18,19,20-tetranorprostaglandin F_{2α},
16-(3,4-dichlorophenoxy)-15-deoxy-15-fluoro-3-oxa-17,18,19,20-tetranorprostaglandin F_{2α},
16-(3-chlorophenoxy)-15-deoxy-15-fluoro-3-oxa-17,18,19,20-tetranorprostaglandin F_{2α},
N-methanesulfonyl-16-(3,5-dichlorophenoxy)-15-deoxy-15-fluoro-17,18,19,20-tetranorprostaglandin F_{2α} carboxamide,
N-methanesulfonyl-16-(3,4-dichlorophenoxy)-15-deoxy-15-fluoro-17,18,19,20-tetranorprostaglandin F_{2α} carboxamide,
N-methanesulfonyl-16- (3-chlorophenoxy) -15-deoxy-15-fluoro-17,18,19,20-tetranorprostaglandin F_{2α} carboxamide,
N-methanesulfonyl-16-(3,5-difluorophenoxy)-15-deoxy-15-fluoro-17,18,19,20-tetranorprostaglandin F_{2α} carboxamide,
N-methanesulfonyl-16-(3,4-difluorophenoxy)-15-deoxy-15-fluoro-17,18,19,20-tetranorprostaglandin F_{2α} carboxamide,
N-methanesulfonyl-16-(3-fluorophenoxy)-15-deoxy-15-fluoro-17,18,19,20-tetranorprostaglandin F_{2α} carboxamide,
N-methanesulfonyl-16-[3,5-bis(trifluoromethyl)phenoxy]-15-deoxy-15-fluoro-17,18,19,20-tetranorprostaglandin F_{2α} carboxamide,
N-methanesulfonyl-16-[3,4-bis(trifluoromethyl)phenoxy]-15-deoxy-15-fluoro-17,18,19,20-tetranorprostaglandin F_{2α} carboxamide,
N-methanesulfonyl-16-[3-(trifluoromethyl)phenoxy]-15-deoxy-15-fluoro-17,18,19,20-tetranorprostaglandin F_{2α} carboxamide,
N-methanesulfonyl-16-phenyl-15-deoxy-15-fluoro-17,18,19,20-tetranorprostaglandin F_{2α} carboxamide,
N-methanesulfonyl-16-(3-methylphenyl)-15-deoxy-15-fluoro-17,18,19,20-tetranorprostaglandin F_{2α} carboxamide,
N-methanesulfonyl-16-[3-(trifluoromethyl)phenyl]-15-deoxy-15-fluoro-17,18,19,20-tetranorprostaglandin F_{2α} carboxamide,
N-methanesulfonyl-16-phenyl-15-deoxy-15-fluoro-17,18,19,20-tetranorprostaglandin F_{2α} carboxamide and
N-methanesulfonyl-17-phenyl-15-deoxy-15-fluoro-18,19,20-trinorprostaglandin F_{2α} carboxamide.

The fluorine-containing prostaglandin derivative of the formula (1) has asymmetric carbon atoms in its structure and thus has various stereoisomers and optical isomers. The fluorine-containing prostaglandin derivatives of the present invention include all of such stereoisomers, optical isomers and their mixtures.

In respect of biological activities, the compounds of the present invention are comparable to the naturally occurring PGF_{2α} in the effect of lowering intraocular pressure and better in irritation of the eye and influences on the ocular tissues such as the cornea, the iris and the conjunctiva. Further, they are unlikely to decompose through metabolic processes such as hydrolysis and oxidation and stable in the body. They also easily penetrate the cornea and are easily absorbed by the eye. For these reasons, they are very useful as medicines. Therefore, the medicine of the present invention is effective as a therapeutic agent, particularly for glaucoma or ocular hypertension.

The medicine for an eye disease of the present invention is a pharmaceutical containing a compound of the present invention as an active ingredient and typically applied to the eye, for example, in drops. As its dosage forms, external preparations such as eye drops and ophthalmic ointments and injections are mentioned, and the compounds of the present invention are formulated by using common techniques. For example, in the case of eye drops, isotonicities such as sodium chloride and concentrated glycerine, buffering agents such as sodium phosphate and sodium acetate, surfactants such as polyoxyethylene sorbitane monooleate (hereinafter referred to as polysorbate 80), polyoxyl 40 stearate and polyoxyethylene hydrogenated castor oil, stabilizers such as sodium citrate and sodium edetate and antiseptics such as benzalkonium chloride and paraben are optionally used to prepare the medicine of the present invention. The pH should be within a range acceptable for ocular medicines and is preferably from 4 to 8.

Although the dose depends on the condition and the age of the patient and the dosage form, in the case of an ophthalmic solution, it is applied to the eye at a concentration of 0.001 to 5% (w/v), preferably from 0.01 to 0.5% (w/v) once or a couple of times a day.

### EXAMPLES

Now, the present invention will be described in further detail with reference to Examples. However, the present invention is by no means restricted to such specific Examples. In Examples 1 to 18, fluorine-containing prostaglandin derivatives of the present invention and their intermediates were prepared. In Examples 7 to 10 and 16 to 20, fluorine-containing prostaglandin derivatives of the formula (1) were prepared. Example 21 illustrates formulations of the medicine of the present invention. In Example 22, fluorine-containing prostaglandin derivatives of the formula (1) were evaluated as medicines for eye disease. Hereinafter, THF means tetrahydrofuran.

### EXAMPLE 1

### Preparation of methyl 7-[(1 R,2S,3R,5S)-[2-(t-butyldimethylsilyloxy)methyl-5-hydroxy-3-(2-tetrahydropyranyloxy)] cyclopentyl]hexa-5-enoate

To (1S,5R,6S,7R)-2-oxa-7-(2-tetrahydropyranyloxy)-6-(t-butyldimethylsilyloxy)bicyclo[3.3.0]octan-3-one (3.71 g) in toluene (30 mℓ), diisobutylaluminium hydride in toluene (1M, 12 mℓ) was added at -78°C, and the resulting mixture was stirred for 30 minutes and extracted with ethyl acetate after addition of saturated aqueous sodium chloride. Removable of the solvent by evaporation afforded a crude lactol.

4-Carboxybutyltriphenylphosphonium bromide (17.7 g) suspended in THF (160 mℓ) was stirred together with 8.98 g of potassium t-butoxide at room temperature for 1 hour. The crude lactol in THF (20 mℓ) was added, and the resulting mixture was stirred at room temperature for 1 hour. The reaction was quenched with water, and the aqueous layer was extracted with t-butyl methyl ether after acidification. Removal of the solvent by evaporation afforded 8.11 g of a crude carboxylic acid.

To a solution of 8.11 g of the carboxylic acid in dimethyl sulfoxide (100 mℓ) were added 15 mℓ of diisopropylethylamine and 10 mℓ of iodomethane. The mixture was stirred for 1 hour. After addition of water, the mixture was extracted with hexane:ethyl acetate (1:1). Purification by silica gel column chromatography (hexane/ethyl acetate 30/1 - 5/1) afforded 7.31 g of the title compound (yield 43%).

¹H NMR(CDCl₃):δ 0.03(s,6H),0.88(s,9H),1.5-2.5(m,18H),3.5-3.6(m,2H),3 .66(s,3H),3.7-3.9(m,2H),4.1-4.2 (m,2H),4.69(m,1H),5.3-5.4(m,2H).

### EXAMPLE 2

### Preparation of methyl 7-[(R,2S,3R,5S)-[2-hydroxymethyl-5-acetoxy-3-(2-tetrahydropyranyloxy)]cyclopentyl]hexa-5-enoate

2.01 g of the silyl ether prepared in Example 1 was dissolved in 8 mℓ of pyridine. To the solution were added 8 mℓ of acetic anhydride and 5 mg of dimethylaminopyridine, and the mixture was stirred at room temperature for 1 hour. After addition of saturated aqueous sodium bicarbonate, it was extracted with hexane-ethyl acetate (1:1), and the extract was dried over anhydrous sodium sulfate. Removal of the solvent by evaporation afforded 2.41 g of an acetate. The acetate was dissolved in 15 mℓ of THF and stirred together with tetrabutylammonium fluoride (1 M THF solution, 9.54 mℓ) at room temperature for 18 hours. Purification by column chromatography (hexane/ethyl acetate 5/1 - 1/1) after concentration afforded 1.54 g of the title compound (yield 91%).

¹H NMR(CDCl₃):δ 1.5-2.4(m,18H),2.04(s,3H),3.5-3.6(m,2H),3.67(s,3H), 3,8-4.2(m,3H),4.55-4.71(m,1H), 5.04(m,1H),5.37(m,2H).

### EXAMPLE 3

### Preparation of methyl 7-[(1 R,2R,3R,5S)-[2-formyl-5-acetoxy-3-(2-tetrahydropyranyloxy)]cyclopentyl]hexa-5-enoate

To 1.52 g of the alcohol prepared in Example 2 were added 30 mℓ of benzene, 0.68 mℓ of pyridine, 0.088 me of trifluoroacetic acid, 9 me of dimethyl sulfoxide and 3.37 g of dicyclohexylcarbodiimide, and the mixture was stirred at room temperature for 3 hours. Insoluble materials in the resulting mixture was filtered off and washed with ethyl acetate. To the filtrate was added water and extracted with ethyl acetate. The extracts were combined, dried and evaporated to afford 2 g of the crude aldehyde.

### EXAMPLE 4

### Preparation of 16-(3-methylphenyl)-15-deoxy-15-oxo-9-acetyl-11-(2-tetrahydropyranyl)-17,18,19,20-tetranorprostaglandin F_{2α} methyl ester

To sodium hydride (60% dispersion in mineral oil, 85 mg) suspended in 1,2-dimethoxyethane (85 mℓ), dimethyl 2-oxo-3-(3-methylphenyl)propylphosphonate (548 mg) in 1,2-dimethoxyethane (36 mℓ) was added under cooling with ice. After 1 hour of stirring, the aldehyde (2 g) prepared in Example 3 in 1,2-dimethoxyethane (36 mℓ) was added. The resulting mixture was stirred at room temperature for 12 hours, poured into saturated aqueous sodium chloride and extracted with ethyl acetate, and the extract was dried and evaporated. Purification by silica gel column chromatography (hexane/ethyl acetate 3/1) afforded 0.96 g of the title compound.

¹H NMR(CDCl₃) : δ 1.3-2.8(m,24H),3.2-4.1(m,5H),3.66(s,3H),4.45(m,1H), 5.06(m,1H),5.19-5.36(m,2H),6.26 (m,1H),6.78(m,1H),7.0-7.2(m,4H).

### EXAMPLE 5

### Preparation of (15RS)-16-(3-methylphenyl)-9-acetyl-11-(2-tetrahydropyranyl)-17,18,19,20-tetranorprostaglandin F_{2α} methyl ester

To the enone (815 mg) prepared in Example 4 in methanol (30 mℓ), 868 mg of cerium trichloride heptahydrate was added. After having been cooled to -78°C, to the resulting mixture was added 88 mg of sodium borohydride. The mixture was stirred at -78°C for 30 minutes, poured into saturated aqueous sodium bicarbonate and extracted with ethyl acetate. Purification by silica gel column chromatography (hexane/ethyl acetate 3/1) afforded 0.80 g of the title compound.

¹H NMR(CDCl₃) : δ 1.4-2.6(m, 25H),2.70-2.88(m,2H),3.34-4.04(m,3H),3.66 (s, 3H), 4.35 (m, 1H), 4.59 (m, 1H), 5.04 (m, 1H), 5.26-5.38 (m, 2H), 5.49-5.78 (m, 2H), 7.0-7,2(m, 4H).

### EXAMPLE 6

### Preparation of (15RS)-15-deoxy-15-fluoro-16-(3-methylphenyl)-9-acetyl-11-(2-tetrahydropyranyl)-17,18,19,20-tetranorprostaglandin F_{2α} methyl ester

To the alcohol (103 mg) prepared in Example 5 in methylene chloride (10 mℓ), 132 mg of morpholinosulfur trifluoride was added at -78°C. After 1 hour of stirring, the resulting mixture was poured into saturated aqueous sodium bicarbonate and extracted with ethyl acetate. Purification by silica gel column chromatography (hexane/ethyl acetate 5/1) afforded 89 mg of the title compound.

¹H NMR(CDCl₃) : δ 1.4-2.6(m, 24H), 2.8-3.1 (m, 2H), 3.3-4.0(m, 3H), 3.65(s, 3H), 4.55(m, 1H), 4.9-5.4(m, 4H), 5.56-5.78(m, 2H), 7.0-7.2(m, 4H).

¹⁹F NMR(CDCl₃) : -171.4(m), -170.4(m).

### EXAMPLE 7

### Preparation of (15RS)-15-deoxy-15-fluoro-16-(3-methylphenyl)-9-acetyl-17,18,19,20-tetranorprostaglandin F_{2α} methyl ester

The fluoride (72 mg) prepared in Example 6 was dissolved in 2 mℓ of methanol. To the solution was added 3 mg of p-toluenesulfonic acid monohydrate, and the mixture was stirred at room temperature for 2 hours. The reaction was quenched with saturated aqueous sodium bicarbonate, and the mixture was extracted with ethyl acetate. The extracts were washed with water and dried over anhydrous sodium sulfate. Removal of the solvent by evaporation under reduced pressure afforded 65 mg of the title compound.

¹H NMR(CDCl₃):δ 1.5-2.5(m,19H),2.83-3.12(m,2H),3.65(s,3H),3.79(m,1H),4.9-5.8(m,6H),6.9-7.2(m,4H).

¹⁹F NMR(CDCl₃):-171.6(m),-170.9(m).

### EXAMPLE 8

### Preparation of (15RS)-15-deoxy-15-fluoro-16-(3-methylphenyl)-17,18,19,20-tetranorprostaglandin F_{2α} methyl ester

To the acetate (65 mg) prepared in Example 7 in methanol (1.7 mℓ), sodium methoxide (28% methanol solution, 0.074 mℓ) was added under cooling with ice, and the resulting mixture was stirred at room temperature for 1 hour. After addition of saturated aqueous sodium bicarbonate, it was extracted with chloroform. The extracts were combined, dried and evaporated. Purification by silica gel column chromatography (hexane/ethyl acetate 2/1) afforded 49 mg of the title compound.

¹H NMR(CDCl₃):δ 1.4-2.5(m,17H),2.84-3.07(m,2H),3.66(s,3H),3.90(m, 1H),4.17(m,1H),5.06(m,1H),5.37(m, 2H),5.50-5.68(m,2H),6.97-7.30(m,4H).

¹⁹F NMR(CDCl₃):-171.0(m),-170.6(m).

### EXAMPLE 9

### Preparation of (15RS)-15-deoxy-15-fluoro-16-[3-(trifluoromethyl)phenyl]-17,18,19,20-tetranorprostaglandin F_{2α} methyl ester

The title compound was prepared from methyl 7-[(1 R,2R,3R,5S)-[2-formyl-5-acetoxy-3-(2-tetrahydropyranyloxy)]cyclopentyl]hexa-5-onoate prepared in Example 3 and dimethyl 2-oxo-3-(3-trifluoromethyl)phenylpropylphosphonate in the same manners as in Examples 4, 5, 6, 7 and 8.

¹H NMR(CDCl₃) : δ 1.4-2.5(m, 14H), 2.9-3.2(m, 2H), 3.66(s, 3H), 3.93(m, 1 H), 4.19(m, 1H), 5.08(m, 1H), 5.3-5.5(m, 2H), 5.55-5.70(m, 2H), 7.35-7.60(m, 4H).

¹⁹F NMR(CDCl₃):-172.1(m),-171.7(m),-63.1(s).

### EXAMPLE 10

### Preparation of 16-(3,5-dichlorophenoxy)-15-deoxy-15-fluoro-17,18,19,20-tetranorprostaglandin F_{2α} isopropyl ester

The methyl ester of the PGF_{2α} identified above was prepared by treating methyl 7-[(1R,2R,3R,5S)-[2-formyl-5-acetoxy-3-(2-tetrahydropyranyloxy)]cyclopentyl]hexa-5-enoate and dimethyl [3-(3,5-dichlorophenoxy)-2-oxopropyl] phosphonate in the same manners as in Examples 4, 5, 6, 7 and 8 and separating the 15-isomer by using a column. Hydrolysis of the methyl ester with 1 N NaOH and subsequent isopropylation of the resulting carboxylic acid with 2-iodopropane in acetone in the presence of diazabicycloundecene gave the title compound.

¹H NMR(CDCl₃) : δ 1.23 (d, J=6.4Hz, 6H), 1.57-1.75(m, 3H), 1.84(d, J=13.7Hz, 1H), 2.07-2.46(m, 8H), 4.00-4.06(m, 3H), 4.10-4.22(m, 1H), 4.79-5.04(m, 1H), 5. 13-5.28(m, 1H), 5.37-5.42(m, 2H), 5.70(ddd, J=6.3, 12.2, 15.6Hz, 1H), 5.84(ddd, J=2.9, 8.8, 15.6Hz, 1H), 6.83(d, J=2.0Hz, 2H), 6.98(t, J=2.0Hz, 1H).

19^{F} NMR(CDCl₃):-180.4(m).

### EXAMPLE 11

### Preparation of (1S,5R,6R,7R)-2-oxa-7-benzoyloxy-6-[(1E)-4-(3,5-dichlorophenoxy)-3-oxo-1-butenyl]-bicyclo[3.3.0] octan-3-one

To sodium hydride (60% dispersion in mineral oil, 8.02 g) suspended in THF (1000 mℓ), dimethyl 2-oxo-3-(3,5-dichlorophenoxy)-propylphosphonate (70.0 g) in THF (750 mℓ) was added under cooling with ice. After 1 hour of stirring, (1 S,5R,6R,7R)-6-formyl-7-benzoyloxy-2-oxa-bicyclo[3.3.0]octan-3-one (52.4 g) in THF (750 mℓ) was added. The resulting mixture was stirred at 0°C for 1 hour and at room temperature for 1 hour, then adjusted to pH 5 - 6 with acetic acid, poured into water and extracted with ethyl acetate. The extracts were washed with saturated aqueous sodium chloride, dried and evaporated. Purification by silica gel column chromatography (hexane/ethyl acetate 2/1 - 3/2) afforded 53.4 of the title compound.

¹H NMR(CDCl₃):δ 2.31 (ddd, J=2.0, 4.9, 15.6Hz, 1 H), 2.51 (m, 1 H), 2.65(dt, J =6.5, 15.6Hz, 1 H), 2.87-2.98(m, 3H), 4. 67(s, 2H), 5.11(dt, J=2.0,6.5Hz, 1H), 5.3 5 (m, 1H), 6.54(d, J=16.1Hz, 1H), 6.77(d, J=2.0Hz, 2H), 6.92(dd, J=7.8, 16.1 Hz, 1H), 6. 99(t, J=2.0Hz, 1H), 7.45(m, 2H), 7.60(m, 1 H), 7.98(m,2H).

### EXAMPLE 12

### Preparation of (1S,5R,6R,7R)-2-oxa-7-benzoyloxy-6-[(1E, 3S)-3-hydroxy-4-(3,5-dichlorophenoxy)-1-butenyl]-bicyclo [3.3.0]octan-3-one

53.3 g of the enone prepared in Example 11 was dissolved in a solvent mixture of methylene chloride (250 mℓ)-methanol (500 mℓ), and 16.85 g of cerium trichloride heptahydrate was added. The resulting mixture was cooled to -78°C and stirred together with 3.41 g of sodium borohydride for 2.5 hours. After pH adjustment to about 3 with 1 N hydrochloric acid, it was once concentrated, then diluted with ethyl acetate and washed with saturated aqueous sodium chloride and 3% aqueous citric acid. It was dried and evaporated. Purification by silica gel column chromatography (hexane/ethyl acetate 3/2) afforded 26.2 g of the title compound.

¹H NMR(CDCl₃) : δ 2.27(ddd, J=2.0, 5.9, 15.1Hz, 1H), 2.52(d, J=16.1 Hz, 1H), 2.63(dt, J=6.8, 15.1Hz, 1H), 2.7-2.9(m, 3H), 3.76(dd, J=7.3, 9.3Hz, 1H), 3.90(dd , J=3.4, 9.3Hz, 1H), 4.50(m, 1H), 5.07(dt, J=2.0, 6.3Hz, 1H), 5.28 (dd, J=5.9, 12.2 Hz, 1H), 5.70(dd, J=5.4, 15.6Hz, 1H), 5.80(ddd, J=0.97, 7.8, 15.6Hz,1H), 6.74(d, J=2.0Hz, 1H), 6.97 (t, J=2.0Hz,1 H), 7.44(m, 2H), 7.57(m, 1 H), 7.99(m, 2H).

### EXAMPLE 13

### Preparation of (1S,5R,6R,7R)-2-oxa-7-benzoyloxy-6-[(1E, 3R)-3-fluoro-4-(3,5-dichlorophenoxy)-1-butenyl]-bicyclo [3.3.0]octan-3-one

To 19.2 g of the alcohol prepared in Example 12 in methylene chloride (500 mℓ), 25.0 g of morpholinosulfur trifluoride was added at -78°C. After 1 hour of stirring, the resulting mixture was poured into saturated aqueous sodium bicarbonate and extracted with ethyl acetate. Purification by silica gel column chromatography (hexane/ethyl acetate 3/1) afforded 12.5 g of the title compound.

¹H NMR(CDCl₃) : δ 2.3-3.0(m, 5H), 4,00(m, 2H), 5.08-5.31(m, 3H), 5.73-5.88 (m, 2H), 6.74(m, 2H), 6.97(m, 1H), 7.42(m, 2H), 7.56(m, 1H), 7.96(m, 2H).

¹⁹F NMR(CDCl₃):-182.8(m).

### EXAMPLE 14

### Preparation of (1S,5R,6R,7R)-2-oxa-7-hydroxy-6-[(1E,3R)-3-fluoro-4-(3,5-dichiorophenoxy)-1-butenyl]-bicydo[3.3.0] octan-3-one

12.5 g of the fluoride prepared in Example 13 was dissolved in 120 me of methanol. To the solution was added 3.04 g of potassium carbonate, and the mixture was stirred at room temperature for 2 hours. The resulting mixture was adjusted to pH about 7 with 2N hydrochloric acid and extracted with ethyl acetate after addition of water. Purification by silica gel column chromatography (hexane/ethyl acetate 3/2 - 1/3) afforded 9.7 9 of the title compound.

¹H NMR(CDCl₃) : δ 2.00(dd, 2.7, 6.6Hz, 1H), 2.43-2.82 (m, 6H), 4.03-4.12(m, 3H), 4.95(m, 1H), 5.13-5.29(m, 1H), 5.70-5.84(m, 2H), 6.82(m, 2H), 6.98(m, 1H).

¹⁹F NMR(CDCl₃):-182.4(m).

### EXAMPLE 15

### Preparation of (1S,5R,6R,7R)-2-oxa-3,7-dihydroxy-6-[(1E,3R)-3-fluoro-4-(3,5-dichlorophenoxy)-1-butenyl]-bicyclo [3.3.0]octane

To (1S,5R,6R,7R)-2-oxa-7-hydroxy-6-[(1E,3R)-3-fluoro-4-(3,5-dichlorophenoxy)-1-butenyl]-bicyclo[3.3.0]octan-3-one (9.6 g) prepared in Example 14 in THF (250 mℓ), diisobutylaluminium hydride in toluene (1M, 97 me) was added at -78°C, and the resulting mixture was stirred for 1 hour and extracted with ethyl acetate after addition of water (150 mℓ) and 1N hydrochloric acid (150 mℓ). Removal of the solvent by evaporation after drying afforded 9.4 g of a crude preparation of the title compound.

¹H NMR(CDCl₃):δ 1.99-2.83(m,8H),3.92-4.10(m,3H),4.63(m,1H),5.14-5.27(m,1H),5.56-5.86(m,3H),6.82(m, 2H),6.99(m,1H).

¹⁹F NMR(CDCl₃):-181.1(m).

### EXAMPLE 16

### Preparation of 16-(3,5-dichlorophenoxy)-15-deoxy-15-fluoro-17,18,19,20-tetranorprostaglandin F_{2α} isopropyl ester

44.0 g of 4-carboxybutyltriphenylphosphonium bromide suspended in THF (500 me) was stirred together with 22.3 g of potassium t-butoxide at room temperature for 1.5 hours. 9.40 g of the crude lactol prepared in Example 15 in THF (100 mℓ) was added, and the resulting mixture was stirred at room temperature for 14 hours. The reaction was quenched with water, and the reaction mixture was washed with diethyl ether. After acidification, the aqueous layer was extracted with ethyl acetate. Removal of the solvent by evaporation afterdrying afforded 5.90 g of a crude carboxylic acid. A solution of 5.90 g of the resulting carboxylic acid in acetone (50 mℓ) was stirred together with 11.7 g of diazabicycloundecene and 14.2 g of 2-iodopropane for 3.5 hours. After addition of aqueous sodium bicarbonate, the mixture was extracted with ethyl acetate, and the extract was washed with saturated aqueous sodium chloride, 3% aqueous citric acid and aqueous sodium bicarbonate, and dried and concentrated. Purification by silica gel column chromatography (hexane/ethyl acetate 2/3) afforded 4.1 g of the title compound.

¹H NMR(CDCl₃):δ 1.23(d, J=6.4Hz, 6H), 1.57-1.75(m, 3H), 1.84(d, J=13.7Hz 1 H), 2.07-2.46(m,8H),4.00-4.06 (m, 3H), 4.10-4.22(m, 1H), 4.99-5.04(m, 1H), 5 .13-5.28(m,1H), 5.37-5.42(m, 2H), 5.70(ddd, J=6.3, 12.2, 15.6Hz, 1H), 5.84(ddd , J=2.9, 8.8, 15.6Hz, 1H), 6.83(d, J=2.0Hz, 2H), 6.98(t, J=2.0Hz, 1H).

¹⁹F NMR(CDCl₃):-180.4(m).

### EXAMPLE 17

### (15RS)-15-Deoxy-15-fluoro-16-[3-methylphenyl]-17,18,19,20-tetranorprostaglandin F_{2α} methyl ester

The title compound was prepared from (1S, 5R, 6R, 7R)-6-formyl-7-benzoyloxy-2-oxa-bicyclo[3.3.0]octan-3-one and dimethyl 2-oxo-3-(3-methylphenyl)propylphosphonate in the same manners as in Examples 11 to 16 (except that iodomethane was used instead of 2-iodopropane for esterification).

¹H NMR(CDCl₃):δ 1.4-2.5(m,17H),2.84-3.07(m,2H),3.66(s,3H),3.90(m,1H),4.17(m,1H),5.06(m,1H),5.37(m, 2H),5.50-5.68(m,2H),6.97-7.30(m,4H).

¹⁹F NMR(CDCl₃):-171.0(m),-170.6(m).

### EXAMPLE 18

### (15RS)-15-Deoxy-15-fluoro-16-[3-(trifluoromethyl)phenyl]-17,18,19,20-tetranorprostaglandin F_{2α} methyl ester

The title compound was prepared from (1S,5R,6R,7R)-6-formyl-7-benzoyloxy-2-oxa-bicyclo[3.3.0]octan-3-one and dimethyl 2-oxo-3-[3-(trifluoromethyl)phenyl]propylphosphonate in the same manners as in Examples 11 to 17.

¹H NMR(CDCl₃):δ 1.4-2.5(m,14H),2.9-3.2(m,2H),3.66(s,3H),3.93(m,1H),4.19(m,1H),5.08(m,1H),5.3-5.5(m, 2H),5.55-5.70(m,2H),7.35-7.60(m,4H).

¹⁹F NMR(CDCl₃):-172.1(m),-171.7(m),-63.1(s).

### EXAMPLE 19

### (15RS)-16-(3-Chlorophenoxy)-15-deoxy-15-fluoro-17,18,19,20-tetranorprostaglandin F_{2α} isopropyl ester

The title compound was prepared from methyl 7-[(1 R,2R,3R,5S)-[2-formyl-5-acetoxy-3-(2-tetrahydropyranyloxy)]cyclopentyl]hexa-5-enoate prepared in Example 3 and dimethyl [3-(3-chlorophenoxy)-2-oxopropyl]phosphonate in the same manners as in Examples 4 to 8.

¹H NMR(CDCl₃) : δ 1.22(d, J=7.3Hz, 6H), 1.5-2.5(m, 12H), 3.9-4.2(m, 4H), 5 .00(m, 1H), 5.1-5.3(m, 1H), 5.40 (m, 1H), 5.65-5.90(m, 2H), 6.81(m, 1H), 6.9-7.0(m, 2H), 7.20(t, J=8.1Hz, 1H).

¹⁹F NMR(CDCl₃) : -180.5(m), -180.9(m).

### EXAMPLE 20

### 16-Phenoxy-15-deoxy-15-fluoro-13,14-dihydro-17,18,19,20-tetranorprostaglandin F_{2α} methyl ester

The title compound was prepared from methyl 7-[(1R,2R,3R,5S)-[2-formyl-5-acetoxy-3-(2-tetrahydropyranyloxy)]cyclopentyl]hexa-5-enoate prepared in Example 3 and dimethyl [3-phenoxy-2-oxopropyl]phosphonate in the same manners as in Examples 11 to 16 provided that the enone prepared in the same manners as in Example 11 was reduced to an allyl alcohol and hydrogenated in the presence of a palladium catalyst into an alcohol, which was used for the reaction in Example 12 and subsequent reactions.

¹H NMR(CDCl₃):δ 1.40-1.47(m,2H),1.60-1.73(m,4H),1.85-2.09(m,4H),2 .11-2.23(m,3H),2.31-2.36(m,3H), 3.66(s,3H),3.95(br s, 1H),4.01-4.12(m,2H), 4.17(br s, 1H), 4.79-4.93(m,1H),5.35-5.47(m,2H),6.91(d,J=8.1Hz,2H),6.9 6(t,J=7.3Hz,1H),7.28(t,J=8.1Hz,2H).

¹⁹F NMR(CDCl₃):-188.1(m).

### EXAMPLE 21 (formulation example)

Typical formulations of an ophthalmic solution and an ophthalmic ointment containing 16-(3,5-dichlorophenoxy)-15-deoxy-15-fluoro-17,18,19,20-tetranorprostaglandin F_{2α} isopropyl ester (hereinafter referred to as Compound A) are given below.

### 1) Ophthalmic solution

| Formulation 1 (Pharmaceutical 1) 10 mℓ | |
|---|---|
| Compound A | 1 mg |
| Concentrated glycerine | 250 mg |
| Polysorbate 80 | 200 mg |
| Sodium dihydrogenphosphate dihydrate | 20 mg |
| Sterilized pure water | appropriate amount |
| 1N hydrochloric acid or 1N sodium hydroxide | appropriate amount |
| pH | 6.0 |

| Formulation 2 (Pharmaceutical 2) 10 mℓ | |
|---|---|
| Compound A | 5 mg |
| Concentrated glycerine | 250 mg |
| Polysorbate 80 | 200 mg |
| Sodium dihydrogenphosphate dihydrate | 20 mg |
| Sterilized pure water | appropriate amount |
| 1N hydrochloric acid or 1N sodium hydroxide | appropriate amount |
| pH | 6.0 |

| Formulation 3 (Pharmaceutical 3) 10 mℓ | |
|---|---|
| Compound A | 10 mg |
| Concentrated glycerine | 250 mg |
| Polysorbate 80 | 200 mg |
| Sodium dihydrogenphosphate dihydrate | 20 mg |
| Sterilized pure water | appropriate amount |
| 1N hydrochloric acid or 1N sodium hydroxide | appropriate amount |
| pH | 6.0 |

| Formulation 4 (Pharmaceutical 4) 10 mℓ | |
|---|---|
| Compound A | 50 mg |
| Concentrated glycerine | 250 mg |
| Polysorbate 80 | 500 mg |
| Sodium dihydrogenphosphate dihydrate | 20 mg |
| Sterilized pure water | appropriate amount |
| 1N hydrochloric acid or 1N sodium hydroxide | appropriate amount |
| pH | 6.0 |

### 2) Ophthalmic ointment

| Formulation 1 (Pharmaceutical 5) 100 g | |
|---|---|
| Compound A | 0.1 g |
| Liquid paraffin | 20 g |
| White soft paraffin | 77.9 g |
| Purified lanolin | 2 g |

The formulation of the ophthalmic solution containing Latanoprost which was used as a comparative compound in the pharmacological tests is shown below.

| Formulation 1 (Reference Pharmaceutical) 10 ml | |
|---|---|
| Latanoprost | 10 mg |
| Concentrated glycerine | 250 mg |
| Polysorbate 80 | 200 mg |
| Sodium dihydrogenphosphate dihydrate | 20 mg |
| Sterilized pure water | appropriate amount |
| 1N hydrochloric acid or 1N sodium hydroxide | appropriate amount |
| pH | 6.0 |

### EXAMPLE 22 (pharmacological tests)

The effect of a compound of the present invention on intraocular pressure and eye irritations caused by it were investigated to find their usefulness as medicines for an ocular disease.

### 1) Effects on intraocular pressure

The effect of a compound of the present invention on intraocular pressure was studied in accordance with the method disclosed in a report of a study on the effect of the tromethamine salt and the isopropyl ester of PGF_{2α} on intraocular pressure in crab-eating macaques (Exp. Eye Res., 61, 677-683 (1995)).

### (Method)

Male crab-eating macaques weighing from 5 to 7.5 kg (5-10 years old) were used in the test. The intraocular pressures were measured just before and 2, 4 and 6 hours after application of the test compound under ketamine anesthasia (5-10 mg/kg, intramuscular administration) by means of an air-puff applanation tonometer.

### (Results)

Table 1 shows the resulting changes in intraocular pressure (average of 8 subjects) with time after application of 20 µℓ of 0.01% (w/v) ophthalmic solution (pharmaceutical 1) and 0.1% (w/v) ophthalmic solution (pharmaceutical 3) containing compound A, in relation to the initial intraocular pressure (the intraocular pressure just before application). The results of application of 0.1% (w/v) ophthalmic solution containing Latanoprost (reference pharmaceutical 1), which is known as a therapeutic agent for glaucoma are also shown in Table 1.

**Table 1**

| | Change in ocular pressure after application (mmHg) | | |
|---|---|---|---|
| | 2 hours | 4 hours | 6 hours |
| Compound A (0.01%) | -0.8 | -1.8 | -3.0 |
| Compound A (0.1%) | -1.0 | -1.4 | -3.6 |
| Latanoprost (0.1%) | -0.6 | -2.6 | -2.3 |

As is evident from Table 1, the intraocular pressure had already started to decrease 2 hours after the application of compound A and had appreciably decreased even 6 hours after the application. Compound A had lowered the intraocular pressure about 1.5 times as much as Latanoprost did 6 hours after application. This proves that the compound of the present invention has a long-lasting effect of lowering intraocular pressure.

### 2) Test on eye irritations

The eye irritation caused by a compound of the present invention was investigated in accordance with the method of Fukui et al. ("Gendai-no-rinsho", 4277-89 (1970)).

### (Method)

Male Japanese white rabbits weighing about 2.5 kg were used in the test. A test compound was applied into the conjunctival sac of a right eye ten times at 30 minute intervals. The conditions of the front and the anterior chamber of the eye were observed just before and 1, 2 and 4 hours after the final application, and the damage to the cornea was observed 4 hours after the final application. Into the conjunctival sac of a left eye, the base only, which was free from the test compound, was applied.
A) Conditions of the front of the eye: Scores were determined on the basis of the following scale of damage to the eye according to the improved Draize method.
   [Scale of damage to the eye]
   The cornea
   a) Opacity
      - No cloud (normal); 0
      - Scattered clouds in pervasive regions (the details of the iris are clearly visible); 1
      - Easily distinguishable translucent regions (details of the iris are not visible so clearly); 2
      - Opaque regions, details of the iris are invisible, and the size of the pupil is barely recognizable; 3
      - Unclear, the iris is invisible; 4
   b) The size of the region of the cornea falling into the above
      - None; 0
      - More than 0 and not more than 1/4; 1
      - More than 1/4 and less than 1/2; 2
      - More than 1/2 and less than 3/4; 3
      - More than 3/4 and all over; 4
   The iris
   - Normal; 0
   - More plicae, stasis, swelling, congestion around the cornea than normal (any or combinations of them), the iris is still responsive to light (positive for slow reaction); 1
   - Irresponsible to light, and bleeding and apparent destruction (any or all of them); 2
   The conjunctiva
   a) Rubor of the eyelid conjunctiva
      - No congestion; 0
      - Slightly reddish mucosae and slight but recognizable vasodilation of the edge of the eyelid; 0.5
      - Clearly abnormal congestion, appreciably red mucosae and remarkable swelling; 1
      - Pretty remarkably red ocular mucosae and slightly unclear peripheral vessels; 2
      - More intense conditions than 2 and pervasive beef-like rubor; 3
   b) Edema of the eyelid conjunctiva
      - Not swollen; 0
      - Slight tendency of edema; 0.5
      - More swollen than normal; 1
      - Apparently swollen together with part of the integument of the eyelid; 2
      - Swollen with about half of the eyelid sagging; 3
      - Swollen with about half to all of the eyelid sagging; 4
   c) Rubor of the supraocular conjunctiva
      - No congestion; 0
      - Slight vasodilation around the cornea; 0.5
      - More remarkable vasodilation; 1
      - Remarkable vasodilation toward the edge of the eyelid or remarkable rubor; 2
   d) Condition of the nictitating membrane
      - No congestion; 0
      - Recognizable vasodilation and tendency of edema; 0.5
      - More appreciable vasodilation and rubor of the edge of the eyelid; 1
      - Extreme vasodilation and remarkable rubor of the entire nictitating membrane; 2
   e) Secretions
      - No secretions; 0
      - A certain amount more than normal (exclusive of small amount of secretions found at the medial angle of the eye of a normal animal); 1
      - Some secretions with a wet eyelid and neighboring fur; 2
      - Some secretions with a wet eyelid and a large area of wet fur around the eye; 3
B) Conditions of the anterior chamber: After the observation of the front of the eye, the inside of the anterior chamber was checked for flares under a slit lamp.
C) Damage to the cornea: After observation of the conditions of the front and anterior chamber of the eye 4 hours after the final application, the cornea was stained with fluorescein, and the remaining dye on the cornea was observed under a slit lamp.

### (Results)

Table 2 shows the scores for the condition of the front of the eye (the average of three subjects) when a 0.01% (w/v) ophthalmic solution (pharmaceutical 3) of compound A was applied in 50 µℓ as an example of the results of the experiment. Further, Table 3 shows the results of application of a 0.1% (w/v) ophthalmic solution of Latanoprost (reference pharmaceutical 1) which is known as a therapeutic agent for glaucoma. Further, to comprehensively evaluate damage to the front of the eye, the sum of the scores was calculated as a total score.

**Table 2**

| | Before application | After 1 hr | After 2 hrs | After 4 hrs |
|---|---|---|---|---|
| Opacity of the cornea | 0 | 0 | 0 | 0 |
| | | | | |
| Opaque fraction of the cornea | 0 | 0 | 0 | 0 |
| | | | | |
| Iridial congestion | 0 | 0 | 0 | 0 |
| | | | | |
| Rubor of eyelid conjunctiva | 0 | 0 | 0.2 | 0 |
| | | | | |
| Edema of eyelid conjunctiva | 0 | 0 | 0 | 0 |
| | | | | |
| Rubor of supraocular conjunctiva | 0 | 0.2 | 0.5 | 0.2 |
| | | | | |
| Condition of nictitating membrane | 0 | 0.2 | 0.2 | 0 |
| | | | | |
| Secretions | 0 | 0 | 0 | 0 |
| | | | | |
| Total score | 0 | 0.4 | 0.9 | 0.2 |

**Table 3**

| | Before application | After 1 hr | After 2 hrs | After 4 hrs |
|---|---|---|---|---|
| Opacity of the cornea | 0 | 0 | 0 | 0 |
| | | | | |
| Opaque fraction of the cornea | 0 | 0 | 0 | 0 |
| | | | | |
| Iridial congestion | 0 | 0 | 0 | 0 |
| | | | | |
| Rubor of eyelid conjunctiva | 0 | 0.5 | 0.5 | 0 |
| | | | | |
| Edema of eyelid conjunctiva | 0 | 0 | 0 | 0 |
| | | | | |
| Rubor of supraocular conjunctiva | 0 | 0.8 | 0.8 | 0.3 |
| | | | | |
| Condition of nictitating membrane | 0 | 0.3 | 0.3 | 0.3 |
| | | | | |
| Secretions | 0 | 0.3 | 0 | 0 |
| | | | | |
| Total score | 0 | 1.9 | 1.6 | 0.6 |

As shown in Table 2, compound A was little irritant to the front of the eye when applied to the eye although slight congestion of the conjunctiva was observed. Further, as shown in Table 2 and Table 3, compound A is apparently less irritant to the front of the eye than Latanoprost and scored in total about 1/5, 1/2 and 1/3 as much as Latanoprost did, 1 hour, 2 hours and 4 hours after application, respectively, and thus always scored less than Latanoprost. On the other hand, when the base only was applied, all the scores for the conditions of the front of the eye were 0.

No flares in the anteriorchamber or no damage to the cornea was observed in any case, which clearly indicates that the compound of the present invention is little irritant to the eye.

The prostaglandin derivatives having a fluorine atom at the 15-position of the present invention, especially those having an esterified carboxylic acid moiety or hydroxyl moiety, are useful as an active ingredient of a medicine. Medicines containing the compounds as an active ingredient are useful as preventive or therapeutic agents for eye diseases. The medicines are excellent medicine for eye diseases having a high pharmacological activity and little side effect.

## Claims

1. A fluorine-containing prostaglandin derivative of the following formula (1) (exclusive of 15-fluoro-15-deoxy PGF_{2α} and 15-fluoro-15-deoxy-PGF_{2α} methyl ester) or a salt thereof: 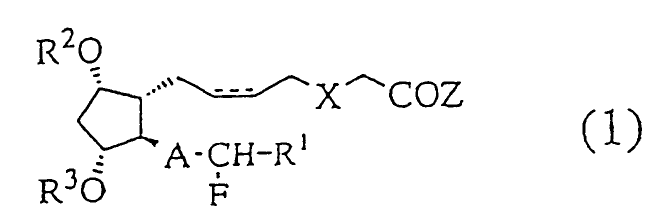 wherein A is an ethylene group, a vinylene group, an ethynylene group, -OCH₂- or -SCH₂-,
R¹ is a substituted or unsubstituted C₃₋₈ alkyl group, a substituted or unsubstituted C₃₋₈ alkenyl group, a substituted or unsubstituted C₃₋₈ alkynyl group, a substituted or unsubstituted C₃₋₈ cycloalkyl group, a substituted or unsubstituted aralkyl group or a substituted or unsubstituted aryloxyalkyl group,
each of R² and R³ which are independent of each other, is a hydrogen atom or an acyl group, or forms a single bond as described later,
X is -CH₂-, -O- or -S-,
Z is -OR⁴, -NHCOR⁵, -NHSO₂R⁶ or -SR⁷, or forms a single bond together with R² or R³,
each of R⁴, R⁵, R⁶ and R⁷ which are independent of one another, is a hydrogen atom, an alkyl group, an alkenyl group, an alkynyl group, a cycloalkyl group, an aryl group or an aralkyl group,
and a dual line consisting of solid and broken lines is a single bond, a cis-double bond or a trans-double bond.

2. The compound according to Claim 1, wherein A is a vinylene group or an ethylene group.

3. The compound according to Claim 1 or 2, wherein X is -CH₂-.

4. The compound according to Claim 1, 2 or 3, wherein both R² and R³ are hydrogen atoms.

5. The compound according to Claim 1, 2, 3 or 4, wherein Z is -OR⁴.

6. The compound according to Claim 1, 2, 3, 4 or 5, wherein R¹ is an aryloxyalkyl group which may have a substituent.

7. The compound according to Claim 1, 2, 3, 4, 5 or 6, wherein R¹ is a 3,5-dichlorophenoxymethyl group, a 3,4-dichlorophenoxymethyl group, a 3-chlorophenoxymethyl group or a phenoxymethyl group.

8. A medicine containing the compound according to Claim 1, 2, 3, 4, 5, 6 or 7 as an active ingredient.

## Patentansprüche

1. Fluor-enthaltendes Prostaglandinderivat mit der folgenden Formel (1) (außer 15-Fluoro-15-deoxy PGF_{2α} und 15-Fluoro-15-deoxy-PGF_{2α}-methylester) oder einem Salz davon: 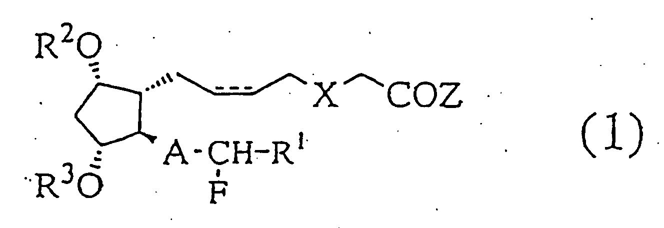 wobei A eine Ethylengruppe, eine Vinylengruppe, eine Ethinylengruppe, -OCH₂- oder -SCH₂- ist,
R¹ eine substituierte oder unsubstituierte C₃₋₈ Alkylgruppe, eine substituierte oder unsubstituierte C₃₋₈ Alkenylgruppe, eine substituierte oder unsubstituierte C₃₋₈ Alkinylgruppe, eine substituierte oder unsubstituierte C₃₋₈ Cycloalkylgruppe, eine substituierte oder unsubstituierte Aralkylgruppe oder eine substituierte oder unsubstituierte Aryloxyalkylgruppe ist,
jedes von R² und R³, die unabhängig voneinander sind, ein Wasserstoffatom oder eine Acylgruppe ist oder eine Einfachbindung, wie nachstehend beschrieben, bildet,
X -CH₂-, -O- oder -S- ist,
Z -OR⁴, -NHCOR⁵-, -NHSO₂R⁶ oder -SR⁷ ist oder eine Einfachbindung zusammen mit R² oder R³ bildet,
jedes von R⁴, R⁵, R⁶ und R⁷, die unabhängig voneinander sind, ein Wasserstoffatom, eine Alkylgruppe, eine Alkenylgruppe, eine Alkinylgruppe, eine Cycloalkylgruppe, eine Arylgruppe oder eine Aralkylgruppe ist, und
eine Doppellinie, bestehend aus durchgezogenen und unterbrochenen Linien, eine Einfachbindung, eine cis-Doppelbindung oder eine trans-Doppelbindung ist.

2. Verbindung nach Anspruch 1, wobei A eine Vinylengruppe oder eine Ethylengruppe ist.

3. Verbindung nach Anspruch 1 oder 2, wobei X -CH₂- ist.

4. Verbindung nach Anspruch 1, 2 oder 3, wobei sowohl R² als auch R³ Wasserstoffatome sind.

5. Verbindung nach Anspruch 1, 2, 3 oder 4, wobei Z -OR⁴ ist.

6. Verbindung nach Anspruch 1, 2, 3, 4 oder 5, wobei R¹ eine Aryloxyalkylgruppe ist, die einen Substituenten aufweisen kann.

7. Verbindung nach Anspruch 1, 2, 3, 4, 5 oder 6, wobei R¹ eine 3,5-Dichlorophenoxymethylgruppe, eine 3,4-Dichlorophenoxymethylgruppe, eine 3-Chlorophenoxymethylgruppe oder eine Phenoxymethylgruppe ist.

8. Medikament, welches die Verbindung gemäß Anspruch 1, 2, 3, 4, 5, 6 oder 7 als einen aktiven Bestandteil enthält.

## Revendications

1. Dérivé de prostaglandine contenant du fluor de formule (1) suivante (à l'exclusion de l'ester méthylique du 15-fluoro-15-désoxy-PGF_{2α} et du 15-fluoro-15-désoxy-PGF_{2α}) ou un sel de celui-ci : 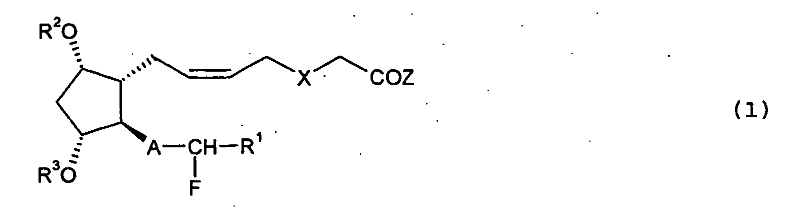 dans laquelle A est un groupe éthylène, un groupe vinylène, un groupe éthynylène, -OCH₂- ou -SCH₂-, R¹ est un groupe alkyle en C₃ à C₈ substitué ou non substitué, un groupe alcényle en C₃ à C₈ substitué ou non substitué, un groupe alcynyle en C₃ à C₈ substitué ou non substitué, un groupe cycloalkyle en C₃ à C₈ substitué ou non substitué, un groupe aralkyle substitué ou non substitué ou un groupe aryloxyalkyle substitué ou non substitué,
chacun de R² et R³, qui sont indépendants l'un de l'autre, est un atome d'hydrogène ou un groupe acyle, ou bien forme une liaison simple comme décrit plus loin,
X est -CH₂-, -O- ou -S-,
Z est -OR⁴, -NHCOR⁵, -NHSO₂R⁶ ou -SR⁷, ou bien forme une liaison simple conjointement avec R² ou R³,
chacun de R⁴, R⁵, R⁶ et R⁷, qui sont indépendants les uns des autres, est un atome d'hydrogène, un groupe alkyle, un groupe alcényle, un groupe alcynyle, un groupe cycloalkyle, un groupe aryle ou un groupe aralkyle,
et la ligne double constituée d'une ligne continue et d'une ligne interrompue est une liaison simple, une double liaison cis ou une double liaison trans.

2. Composé selon la revendication 1, dans lequel A est un groupe vinylène ou un groupe éthylène.

3. Composé selon la revendication 1 ou 2, dans lequel X est -CH₂-.

4. Composé selon la revendication 1, 2 ou 3, dans lequel tant R² que R³ sont des atomes d'hydrogène.

5. Composé selon la revendication 1, 2, 3 ou 4, dans lequel Z est -OR⁴.

6. Composé selon la revendication 1, 2, 3, 4 ou 5, dans lequel R¹ est un groupe aryloxyalkyle qui peut avoir un substituant.

7. Composé selon la revendication 1, 2, 3, 4, 5 ou 6, dans lequel R¹ est un groupe 3,5-dichlorophénoxyméthyle, un groupe 3,4-dichlorophénoxyméthyle, un groupe 3-chlorophénoxyméthyle ou un groupe phénoxyméthyle.

8. Médicament contenant le composé selon la revendication 1, 2, 3, 4, 5, 6 ou 7 à titre d'ingrédient actif.
